# FASCICULE DE BREVET EUROPEEN

(11) **EP 4 098 290 B1**
(45) Date de publication et mention de la délivrance du brevet: **15.10.2025**
(21) Numéro de dépôt: 22186084.4
(22) Date de dépôt: 29.10.2013
(51) Int. Cl.: A61M 1/14, A61M 1/16, A61M 1/34, A61M 60/109, A61M 60/279, A61M 60/37, A61M 60/441, F16K 31/528, F16K 7/16, A61M 1/36

(54) **DISPOSITIF DE TRAITEMENT EXTRA CORPOREL DU SANG**
GERÄT ZUR EXTRAKORPORALEN BEHANDLUNG VON BLUT
DEVICE FOR TREATING BLOOD OUTSIDE THE BODY

(30) Priorité: 29.10.2012 WO PCT/IB2012/055972
(43) Date de publication de la demande: 07.12.2022
(62) Demande divisionnaire de: 13821164.4
(73) Titulaire: Debiotech S.A., 1004 Lausanne (CH)
(72) Inventeur: THIEBAUD, Pierre, 2088 Cressier (CH); MAGNENAT, Olivier, 1004 Lausanne (CH); CUENI, Reto, 1053 Cugy (CH); NEFTEL, Frédéric, 1005 Lausanne (CH)
(74) Mandataire: Weihs, Bruno Konrad

(56) Documents cités:
- EP-A1- 0 611 227
- WO-A1-2012/127298
- US-A- 5 447 417
- US-A- 6 036 459
- US-A1- 2004 167 457
- US-A1- 2012 022 441
- US-A1- 2012 175 296

## Description

### Domaine de l'invention

L'invention concerne un dispositif d'entrainement pour une pompe péristaltique d'un dispositif médical.

### Etat de la technique

Il existe plusieurs causes possibles pour qu'une personne soit atteinte d'une insuffisance rénal où l'arrêt temporaire/définitif et partiel/total contraint cette personne à utiliser des dispositifs extra corporel qui se substitue totalement ou partiellement à ses propres reins. La dialyse est une technique de purification du sang. Elle permet, à un patient souffrant d'une telle maladie, d'éliminer les impuretés telles que l'urée et l'excès d'eau de l'organisme qui auraient été habituellement éliminé par des reins fonctionnant normalement.

Il est possible de distinguer deux types d'insuffisance rénal liés à la durée de la maladie et nécessitant des soins pouvant être sensiblement différents : l'insuffisance rénale chronique et l'insuffisance rénale aigüe. Une insuffisance rénale chronique contraint le patient à suivre un traitement à vie et à une certaine fréquence. Pour cela, le patient peut effectuer ce traitement dans un centre médical ou à domicile à l'aide d'un appareil à dialyse péritonéale ou à hémodialyse. Une insuffisance rénale aigüe est une maladie temporaire où le patient a besoin d'un appareil pour remplacer temporairement les fonctions de ses reins. Dans ce cas, le patient suit une thérapie d'épuration extra-rénale continue. La dialyse péritonéale et la thérapie d'épuration extra-rénale continue sont très différentes ainsi que les techniques et/ou dispositifs utilisés :
- La dialyse péritonéale utilise le péritoine du patient qui est la membrane naturelle qui enveloppe les parois du ventre et les organes situés dans le ventre (foie, intestins, etc..). La membrane péritonéale dispose d'une très grande surface et elle comporte de très nombreux vaisseaux sanguins. Elle joue ainsi le rôle de filtre naturel. De nombreux brevets divulguent des systèmes permettant d'effectuer des dialyses péritonéales dont certains utilisant des cassettes (EP 1 648 536 A2, EP 0 471 000 B1, EP 1 195 171 B1, EP 1 648 536 B1) pour l'injection et le retrait du fluide dans le péritoine du patient.

- La thérapie d'épuration extra-rénale continue (appelé CRRT en anglais : Continuous Renal Replacement Therapy) est un procédé nécessitant d'extraire en continue le sang du patient, de le traiter grâce à un filtre, généralement un dialyseur puis de réinjecter le sang traité au patient. Deux grands principes sont utilisés grâce au filtre :
   ∘ La diffusion permet le passage de molécules de soluté à travers une membrane semi-perméable selon un gradient de concentration. Les solutés vont du milieu le plus concentré (le sang) vers le milieu le moins concentré (le dialysat) pour se répartir de façon uniforme de part et d'autre de la membrane du filtre.
   ∘ La convection permet le transfert simultané de l'eau et de son contenu en solutés au travers de la membrane semi perméable, grâce au gradient de pression hydrostatique transmembranaire. Ainsi, les solutés vont du milieu où la pression est la plus forte (compartiment du sang) vers le milieu où la pression est la plus faible (compartiment du dialysat).
   Il existe différentes techniques d'épuration extra rénale continue utilisant un seul ou les deux principes : l'ultrafiltration continue lente (SCUF : Slow Continuous Ultra-Filtration), l'hémofiltration veino-veineuse continue (CWH : Continuous Ceno-Venous Hemofiltration), l'hémodialyse veino-veineuse continue (CVVHD: Continuous Veno-Venous Hemodialyse), l'hémodiafiltration veino-veineuse continue (CWHDF : Continuous Veno-Venous Hemofiltration), l'échanges plasmatiques (TPE : Therapeutic Plasma Exchange) et l'hémoperfusion (appelé également Blood Detoxification). A ce jour, aucun dispositif n'est capable de proposer l'ensemble de ces traitements sans une intervention de personnels soignants expérimentés. En outre, ces techniques sont principalement utilisées en milieu de soins intensifs. Malheureusement, ces techniques utilisent des appareils volumineux qui peuvent gêner le bon fonctionnement des autres soins effectués sur le patient. De plus, ces appareils sont complexes, disposent de nombreux consommables et nécessite des changements important pour effectuer les différentes techniques citées ci-dessus. Cela implique que le personnel reçoive une formation spécialisée.

Un exemple de l'état de la technique est connu, par exemple, à partir du document US6036459.

### Description de l'invention

L'invention est définie par les caractéristiques de la revendication indépendante 1. Les revendications dépendantes concernent des modes de réalisation particuliers. Les passages ci-dessous fournissent un contexte supplémentaire afin de mieux comprendre l'invention. Seule l'invention telle que revendiquée fait l'objet d'une protection.

### Description générale de la divulgation

La divulgation concerne de multiples améliorations pour dispositifs médicaux, moyens et/ou méthode utilisé pour des dispositifs médicaux.

La présente demande revendique la priorité de la demande portant le numéro PCT/IB2012/055972, déposée le 29 octobre 2012 au nom de Debiotech.

Un premier aspect de la divulgation est relatif à une unique cassette permettant d'effectuer une ou l'ensemble des différentes techniques d'épuration extra rénale continue : l'ultrafiltration continue lente (SCUF), l'hémofiltration veino-veineuse continue (CWH), l'hémodialyse veino-veineuse continue (CVVHD), l'hémodiafiltration veino-veineuse continue (CVVHDF), l'échanges plasmatiques (TPE) et l'hémoperfusion. Dans un mode de réalisation, le dispositif peut également être utilisé dans le cadre d'une dialyse péritonéale où certains éléments et/ou caractéristiques pourraient ne pas être utilisés ou être utilisés pour d'autres fonctionnalités tel que l'échantillonnage ou autres. Dans mode de réalisation, la cassette est partiellement ou entièrement intégré dans l'appareil de dialyse ou certain de ces éléments font partie dudit appareil (par exemple le système de pompage, les capteurs, le filtre,...) ou sont séparés physiquement de la cassette (par exemple le filtre, moyens d'alimentations, les réservoirs, les capteurs, le moyen de chauffage...) ou sont optionnels. Préférentiellement, ladite cassette et l'appareil de dialyse sont tous deux distincts. La casette est jetable alors que l'appareil est réutilisable. Cela signifie, que la cassette peut être remplacée à chaque traitement (utilisation unique, remplacement de la cassette après chaque utilisation) et que l'appareil peut être utilisé à plusieurs reprises avec différentes casettes. Lesdites cassettes sont conçues pour coopérer physiquement et/ou mécaniquement avec l'appareil et/ou vice-versa. En outre, l'utilisation d'une seule cassette permet de simplifier l'utilisation dudit dispositif, réduire les erreurs des opérateurs grâce à cette simplification, automatiser le traitement sans intervention de personnel soignant, limiter le nombre de type de cassette, simplifier la programmation, permettre l'utilisation du dispositif chez le patient et/ou limiter la place occupée par le dispositif.

Selon un deuxième aspect de la divulgation, le dispositif peut comprendre seulement 3 pompes principales pour effectuer au moins une ou l'ensemble des techniques citées dans l'état de l'art (SCUF, CW, CWH, CWHDF, TPE et l'hémoperfusion).

Selon un mode de réalisation, le dispositif comprend un moyen de filtration du sang, au moins un moyen d'alimentation en liquide, deux tuyaux patient - un tuyau de sortie prélevant le sang à traiter et un tuyau d'entré réinjectant le sang traité audit patient - (ou un seul tuyau en cas de dialyse péritonéale ou un tuyau avec deux lumières distincts), un moyen de récupération du filtrat, trois pompes à fluide, une cassette composée de canaux et de valves de manière à diriger les fluides, et un contrôleur qui commande les pompes, l'ouverture et la fermeture desdites valves en fonction du traitement souhaité.

Ladite casette comprend aux moins une chambre de distribution comprenant un seul canal d'entrée, au moins deux canaux de sortie et aux moins deux chambres de raccordement comprenant au moins deux canaux d'entrée et un canal de sortie. Préférentiellement, ladite chambre de distribution comprend un canal d'entré et trois canaux de sortie commandés par le contrôleur (de façon automatique, programmé et/ou commandé) afin de permettre à un fluide d'être injecté dans le moyen de filtration du sang, dans le sang avant le moyen de filtration du sang (pré dilution) et/ou après le moyen de filtration du sang (post dilution). Grâce à cette chambre de distribution, le dispositif peut effectuer n'importe quel traitement par dialyse sans que le personnel soignant (ou autre spécialiste) ne soit présent pour configurer les branchements spécifiques de chaque traitement.

Ledit système de traitement comprend en outre au moins trois chemins fluidiques. Le premier chemin fluidique relie ledit moyen de filtration du sang au moyen de récupération du filtrat. Il comprend une série de canaux et une pompe dédiée. Le deuxième chemin fluidique est dédié à la circulation du sang. Il comprend au moins deux chambres de raccordement, une série de canaux, ledit moyen de filtration du sang, lesdits tuyaux patient et une pompe dédiée. Le troisième chemin fluidique comprend un moyen d'alimentation en liquide, au moins une pompe dédiée, une série de canaux, une chambre de distribution et, optionnellement, un moyen de chauffage. Ladite chambre de distribution comprend au moins trois canaux de sortie distincts :
- un premier canal de sortie alimentant le deuxième chemin fluidique en amont du moyen de filtration (pour effectuer une pré dilution du sang avant d'être filtré par le moyen de filtration),
- un deuxième canal de sortie alimentant le deuxième chemin fluidique en aval du moyen de filtration (pour effectuer une post dilution du sang après avoir été filtré par le moyen de filtration), et
- un troisième canal de sortie alimentant ledit moyen de filtration.

Dans un mode de réalisation, les deux chambres de raccordement et la chambre de distribution sont alimentées en pression positive par deux pompes placées en amont desdites chambres.

Ladite première chambre de raccordement permet de relier le deuxième chemin fluidique au troisième chemin fluidique en amont du filtre (technique de pré dilution). Elle comprend un canal d'entré provenant du deuxième chemin fluidique, un canal d'entré provenant du troisième chemin fluidique et un canal de sortie permettant au sang de s'écouler en direction du filtre., ledit canal d'entré provenant du troisième chemin fluidique peut comprendre une valve commandée par ledit contrôleur, un restricteur de flux et/ou une pompe.

Ladite deuxième chambre de raccordement permet de relier le deuxième chemin fluidique au troisième chemin fluidique en aval du filtre (technique de post dilution). Elle comprend un canal d'entré provenant du deuxième chemin fluidique suite à son écoulement à travers le filtre, un canal d'entré provenant du troisième chemin fluidique et un canal de sortie en direction du patient.

Selon certains modes de réalisation, l'invention peut en outre comprendre :
- Un moyen d'alimentation pour injecter un anticoagulant dans le deuxième chemin fluidique soit directement dans le tuyau de sortie patient soit le plus en amont dans la cassette grâce à une chambre de raccordement qui permet le mélange de l'anticoagulant avec le sang (fluide du deuxième chemin fluidique), et/ou
- Un moyen d'alimentation pour injecter un produit inhibant l'anticoagulant dans le deuxième chemin fluidique soit directement dans le tuyau d'entrée patient soit le plus en aval dans la cassette grâce à une chambre de raccordement qui permet le mélange dudit produit avec le sang (fluide du deuxième chemin fluidique).

Un troisième aspect de la divulgation est relatif à la structure de la cassette, lorsqu'un chemin fluidique (provenant par exemple d'un moyen d'alimentation en liquide) se relie à un autre chemin fluidique, la cassette comprend préférentiellement une chambre de raccordement permettant l'intersection desdits deux chemins fluidiques. Cette chambre de raccordement peut ainsi permettre de mélanger les fluides provenant desdits chemins fluidiques. En outre, au moins un desdits canaux d'entrée de ladite chambre de raccordement peut comprendre une valve, afin que le contrôleur puisse sélectionner le ou les fluides déterminés qui s'écoulera(ont) dans la chambre de raccordement en fonction du traitement souhaité, programmé ou commandé.

Selon un quatrième aspect de la divulgation, un moyen de chauffage se situe dans le troisième chemin fluidique entre la pompe principale du dialysat et la chambre de distribution. Ledit moyen de chauffage est préférentiellement une poche souple alimentée en pression positive par ladite pompe. Dans un mode de réalisation, une deuxième pompe est localisée entre la chambre de distribution et une chambre de raccordement. Préférentiellement, la chambre de distribution est raccordée à une première et deuxième chambre de raccordement. La première chambre de raccordement permet d'effectuer une pré-dilution du fluide s'écoulant dans le deuxième chemin fluidique (c'est à dire dilution du sang avant le moyen de filtrage) alors que la deuxième chambre de raccordement permet une post dilution du fluide dudit deuxième chemin fluidique (c'est à dire dilution du sang après le moyen de filtrage). La première pompe est une pompe de précision et elle permet de connaitre avec précision la quantité de fluide qui sera mélangé dans le sang en pré et post dilution. La deuxième pompe est localisée en aval d'une des deux chambres de raccordement, l'autre et/ou les deux chambres de raccordement peut comprendre une valve. Cette deuxième pompe est une pompe de répartition permettant de répartir une quantité déterminée de fluide entre la première et la deuxième chambre de raccordement. Ce type de système peut comprendre une ou plusieurs autre chambre de raccordement. Préférentiellement, le système co,prend un capteur de pression et le moyen de chauffage peut servir de moyen de stockage temporaire.

Pour optimiser le fonctionnement du système de traitement, l'invention divulgue également les éléments suivants qui peuvent être utilisé avec ou sans la cassette décrite précédemment :
- Un moyen et une méthode de calibration des pompes du premier et troisième chemin fluidique,
- Un capteur de pression déporté du chemin fluidique,
- Un actionneur linéaire économique en énergie,
- Un dispositif d'entrainement utilisé pour les pompes péristaltiques,
- Un moyen d'amortissement des pics de pression.

### Moyen et méthode de calibration des pompes et/ou des capteurs du premier et troisième chemin fluidique :

Certaines techniques d'épuration extra rénale continue nécessitent de connaître avec précision la quantité de volume injecté et retiré respectivement du troisième et premier chemin fluidique. Habituellement, les dispositifs comprennent deux balances, une balance dédiée au dialysat et une autre dédiée au filtrat. La présente invention peut comprendre le même système de balance toutefois ces balances sont très sensibles et volumineuses (car elles doivent pouvoir contenir l'ensemble des fluides). D'autres dispositifs disposent de cavités dont la capacité volumique est connue avec précision. Ces cavités sont localisée directement sur le premier et troisième chemin fluidique (une paroi intermédiaire étant utilisée pour ne pas mélanger les deux fluides) et elles sont successivement remplies puis vidées des fluides desdits chemins fluidiques. Lorsque la cavité se remplit de dialysat, la cavité se vide du filtrat précédemment contenue dans ladite cavité et/ou vice versa. Ces dispositifs peuvent renfermer plusieurs de ces cavités. Toutefois contrairement au dispositif de la présente invention, ces dispositifs ne permettent pas un fonctionnement en continu, ils fonctionnent par une succession d'étapes de remplissage et de vidange des fluides contenus dans lesdites cavités.

Selon un cinquième aspect de la divulgation, le système de traitement dispose d'un moyen de calibration desdites pompes afin de connaître avec précision lesdits volumes. Le système comprend :
∘ Au moins deux moyens de mesure volumique (capteur, système de mass flow, pompe volumique, pompe péristaltique, balance,...) , dont un qui mesure la quantité de liquide s'écoulant à travers le troisième chemin fluidique et un qui mesure la quantité de liquide s'écoulant à travers le premier chemin fluidique ;
∘ un moyen de prélèvement du premier et troisième chemin fluidique ;
∘ un moyen commun de mesure des volumes prélevés.

Ledit moyen commun de mesure mesure la quantité de fluide prélevé par le moyen de prélèvement et permet de comparer les volumes prélevés et de calibrer lesdites pompes et/ou lesdits moyens de mesure volumique.

Dans un mode de réalisation, lesdites pompes sont considérées comme lesdits moyens de mesure volumique car chaque actionnement correspond à un volume donné.

Dans un autre mode de réalisation, les capteurs de volumes sont distincts des pompes et mesurent en continu les volumes propulsés par lesdites pompes. En cas de dérive des volumes pompés, le contrôleur peut corriger l'actionnement desdites pompes pour corriger les volumes ou le différentiel de volume.

Un sixième aspect de la divulgation concerne une méthode de calibration de pompes utilisées pour propulser les fluides des premiers et troisièmes chemins fluidiques et/ou des capteurs placé dans lesdits chemins fluidiques. La méthode permet en outre de calibrer lesdites pompes avant et/ou pendant l'exécution du traitement.

### Capteur de pression déporté du chemin fluidique :

Un septième aspect de la divulgation est relatif à un moyen de mesurer la pression d'un fluide. Dans un mode de réalisation, le système de traitement comprend au moins un capteur de pression dans au moins un desdits chemins fluidiques. Le présent document divulgue un système de distribution de fluide (préférentiellement une cassette) qui permet de prélever et/ou de délivrer un fluide FI1 et de mesurer la pression dudit fluide FI1. Le système comprend un corps rigide composé d'au moins un chemin fluidique à travers lequel ledit fluide FI1 s'écoule, d'au moins un canal distinct dudit chemin fluidique. Ledit canal permet de relier ledit chemin fluidique à une zone de mesure. Le système peut comprendre au moins une ouverture recouverte par une membrane flexible formant ladite zone de mesure. La membrane est façonnée pour recevoir un capteur de pression.

Un fluide FI2 différent du fluide FI1 est contenu dans ladite zone de mesure. Le fluide FI2 remplit au moins partiellement la zone de mesure et/ou ledit canal. Ledit fluide FI2 permet de transmettre la pression du fluide FI1 à ladite membrane.

L'objectif de ce canal est que le fluide FI1 ne puisse entrer en contact avec ladite membrane ou que le fluide FI1 mouille au moins partiellement ladite membrane. Ainsi, le canal peut être façonné de manière à ce que ledit fluide FI1 limite, freine et/ou contrôle l'écoulement du fluide FI1 à travers ledit canal. Il a une forme et une longueur permettant cette fonction et/ou peut comprendre un moyen de contenir le fluide FI1 (tel qu'une membrane, un filtre hydrophile, hydrophobe,...). Préférentielement, la zone de mesure est remplit des deux fluides FI1 et FI2 et/ou les fluides FI1 et FI2 sont en contact avec la membrane.

### Actionneur linéaire économique en énergie :

Dans un mode de réalisation, le système de traitement comprend au moins un actionneur linéaire économique en énergie. Le présent document divulgue un principe innovant permettant de commander un actionneur linéaire consommant une faible quantité d'énergie et de contrôler la position d'une valve. Cet actionneur peut être compris dans un système tel que décrit précédemment mais également dans tout dispositif utilisant un actionneur linéaire. En particulier, l'actionneur doit offrir deux positions de base, à savoir valve fermée (où le piston est en une première position) et valve ouverte (où le piston est en une deuxième position). Le piston peut aussi posséder une troisième position correspondant à un état où le piston de l'actionneur est désengagé du pied de la valve.

Pour assurer la fonction d'actionneur linéaire, deux techniques différentes sont habituellement utilisées : l'électro-aimant ou le moteur brushless monté avec une vis sans fin et un écrou. Le principal inconvénient de ces deux techniques est qu'ils consomment de l'énergie pour maintenir une position. La présente invention divulgue un actionneur linéaire comprenant au moins deux positions stationnaires à faible consommation d'énergie et un rapide retour à une position de sécurité. En outre, l'actionneur, divulgué par le présent document, ne consomme aucune ou une très faible quantité d'énergie pour maintenir ses différentes positions.

Le huitième aspect de la divulgation est ainsi relatif à un actionneur linéaire comprenant un moteur électrique rotatif, un piston et des moyens qui sont interposés entre le moteur électrique et le piston transformant le mouvement de rotation du moteur en un déplacement linéaire du piston. Lesdits moyens interposés comprennent au moins une rampe périphérique disposée à l'intérieur dudit piston, au moins un moyen de guidage permettant au piston de guider le mouvement de translation et au moins un moyen d'appui fixé directement ou indirectement au rotor dudit moteur électrique. Ledit moyen d'appui est façonné de manière à coopérer avec ladite rampe périphérique. Dans un mode de réalisation, ledit actionneur comprend en outre au moins un moyen de compression qui exerce une force contre le piston en direction de l'extrémité distale du piston. La rampe comprend au moins un seuil permettant d'obtenir au moins une position stationnaire sans consommer d'énergie. Au moins un seuil est positionné au sommet de ladite rampe.Dans un mode réalisation préféré, ce seuil est suivi d'un passage permettant au piston de se libérer des contraintes exercées par le moyen d'appui.

Selon un neuvième aspect de la divulgation, l'actionneur permet de garantir une pression d'occlusion donnée lorsque la valve est en position fermée. La position de la valve sans contribution de l'actionneur est préférentiellement une position fermée. Pour garantir une bonne occlusion, l'actionneur dispose d'un moyen de compression pour assurer une pression suffisante d'occlusion de la valve contre son siège lorsque le piston est en première position. Ledit moyen de compression permet d'obtenir une troisième position (lorsque le piston n'est pas engagé avec la valve) où le piston se trouve plus éloigné du support de l'actionneur. Le passage de la troisième position à la première position est effectué lorsque le piston s'engage avec la valve. Autrement dit lorsque la cassette est placée dans le dispositif. Ainsi, le moyen de compression contraint le piston à exercer une force initiale contre la valve qui transmet cette force contre le siège de la valve garantissant une occlusion du chemin fluidique lorsque le piston est en première position. . Dans un mode de réalisation, ledit moyen de compression peut être sur le support de l'actionneur ou dans ledit piston. Autrement dit, lorsque ledit piston de l'actionneur est engagé avec le pied de la valve, soit en première position, un moyen de compression assure une précontrainte afin de garantir une bonne occlusion. Ledit moyen de compression peut être monté dans l'actionneur ou sur le support de l'actionneur, ledit moyen de compression permet d'obtenir une troisième position où le piston de l'actionneur est plus éloigné du support de l'actionneur qu'en première et deuxième position. La pression d'occlusion dépend du design de la valve et du dimensionnement du moyen de compression.

### Dispositif d'entrainement utilisé pour les pompes péristaltiques :

L'invention concerne un dispositif d'entrainement utilisé pour une pompe. Lors de l'utilisation dudit système de traitement, la cassette est insérée dans un cycler qui comprend des capteurs, des actionneurs linéaires (pour ouvrir et fermer les valves) et des moyens pour entrainer les galets de la pompe péristaltique. Afin de garantir un bon fonctionnement du système, il est important que l'ensemble des éléments soit correctement alignés (capteur, actionneur, moyens d'actionnement...). Or une différence entre le point centrale théorique de la tête de pompe et le point centrale effectif peut exister. Cette différence crée des problèmes d'alignement des actionneurs et des capteurs respectivement sur les valves et les zones de mesures de la cassette. Ainsi, des moyens de guidage permettent de palier à ce problème d'alignement pour les capteurs et actionneurs mais déporte la différence sur le système de pompage. Ainsi, des contraintes plus ou moins importantes peuvent s'exercer sur les éléments de la pompe, pouvant affecter la précision de la pompe péristaltique. Le phénomène est d'autant plus important lorsque les pompes sont nombreuses.

Afin de relaxer les tolérances de fabrication et de garantir la précision des pompes péristaltiques, l'invention divulgue un dispositif d'entrainement des pompes comprenant un axe flottant entrainé par un moyen d'entrainement fixé à un rotor. L'axe flottant comprend un ensemble solidaire base/couvercle qui forme une cavité à l'intérieur de laquelle ledit moyen d'entrainement est au moins partiellement circonscrit. En outre, ledit moyen d'entrainement comprend un corps rigide façonné de manière à coopérer avec les parois de ladite cavité afin de permettre une liberté restreinte de l'axe flottant par rapport à l'axe dudit rotor. L'axe flottant permet de décaler l'axe du système de pompage afin de minimiser voir éliminer toutes contraintes exercées par l'axe de la pompe sur l'axe de pompage théorique de la cassette.

### Moyen d'amortissement des pics de pression :

Un onzième aspect de la divulgation est relatif à un moyen d'amortissement des pics de pression.

La quantité d'un fluide pompé peut être influencée par les éléments constitutifs du système. Ces éléments peuvent être le mécanisme de pompage, le mécanisme de soupape, les moyens d'alimentation en liquide (tubes, réservoirs,...). En particulier, le mécanisme de pompage d'une pompe péristaltique peut provoquer des variations de pression. Ainsi, une onde de pression se crée et se propage dans le ou les chemins fluidiques à chaque fois que les galets entre en contact avec le tube flexible. Cette propagation est atténuée ou renforcée par plusieurs facteurs tels que : le type de liquide, la longueur du chemin fluidique, les restrictions, le type de matériaux du système, la quantité de liquide délivrée par le mécanisme de pompage, le type de pompe, les caractéristiques de ses composants (tube flexible,...), la pression en aval de la pompe, ...

Une des améliorations de l'invention permet de réduire l'amplitude des pics de pression ainsi que son influence sur la quantité pompée. Cette réduction est réalisée par l'addition au chemin fluidique d'un moyen conçu pour absorber les pics de pression.

Un autre avantage de cette réduction des pics est également d'obtenir une pression plus constante ce qui augmente le confort du patient. Dans ce mode de réalisation, l'amortissement des pics peut s'effectuer en aval de la pompe lors d'une injection vers le patient et en amont lorsque la pompe retire un fluide provenant du patient (en particulier lors de dialyse péritonéal).

Ce moyen d'amortissement peut être une cavité remplit d'un fluide compressible tel que de l'air. Ce moyen d'amortissement peut être unun élément flexible qui est déformé par les pics de pression et revient dans un état d'équilibre. Cet élément flexible peut être par exemple une membrane polymère dans la paroi du chemin fluidique.

### Liste des figures

L'invention sera mieux comprise ci-après au moyen de quelques exemples illustrés. Il va de soi que l'invention ne se limite pas à ces modes de réalisation.
Figure 1 schématise le système de traitement de sang
Figure 2 schématise l'utilisation de la cassette appliquant un traitement d'ultrafiltration continue lente
Figure 3 schématise l'utilisation de la cassette appliquant un traitement d'hémofiltration veino-veineuse continue
Figure 4 schématise l'utilisation de la cassette appliquant un traitement d'hémodialyse veino-veineuse continue
Figure 5 schématise l'utilisation de la cassette appliquant un traitement d'hémodiafiltration veino-veineuse continue
Figure 6 schématise l'utilisation de la cassette appliquant un traitement d'échanges plasmatiques
Figure 7 schématise l'utilisation de la cassette appliquant un traitement d'hémoperfusion
Figure 8 schématise le système avec plusieurs moyens d'alimentation en liquide
Figure 9 schématise le système avec une re-circulation du deuxième chemin fluidique et rejet
Figure 10 schématise le système avec le capteur de référence
Figure 11 montre le corps rigide de la cassette comprenant un chemin fluidique et son canal pour la mesure de pression
Figure 12 montre le corps de la cassette et la membrane qui vient recouvrir la zone de mesure
Figures 13 et 13' schématisent l'emplacement du capteur déporté d'un chemin fluidique
Figure 14 montre une vue éclaté de l'actionneur linéaire
Figure 15 montre une vue en coupe où la valve est couplé au téton/piston
Figure 16 montre deux vues détaillées du piston
Figure 17 expose deux vues en partie découpé du piston en troisième position, valve (non représentée) non couplée
Figure 18 expose deux vues en partie découpé du piston en première position, valve couplée (non représentée)
Figure 19 expose deux vues en partie découpé du piston passant de la première à la deuxième position, valve couplée (non représentée)
Figure 20 montre le piston en deuxième position, valve couplée (non représentée)
Figure 21 montre le piston en deuxième position qui passe instantanément en première position, valve couplée (non représentée)

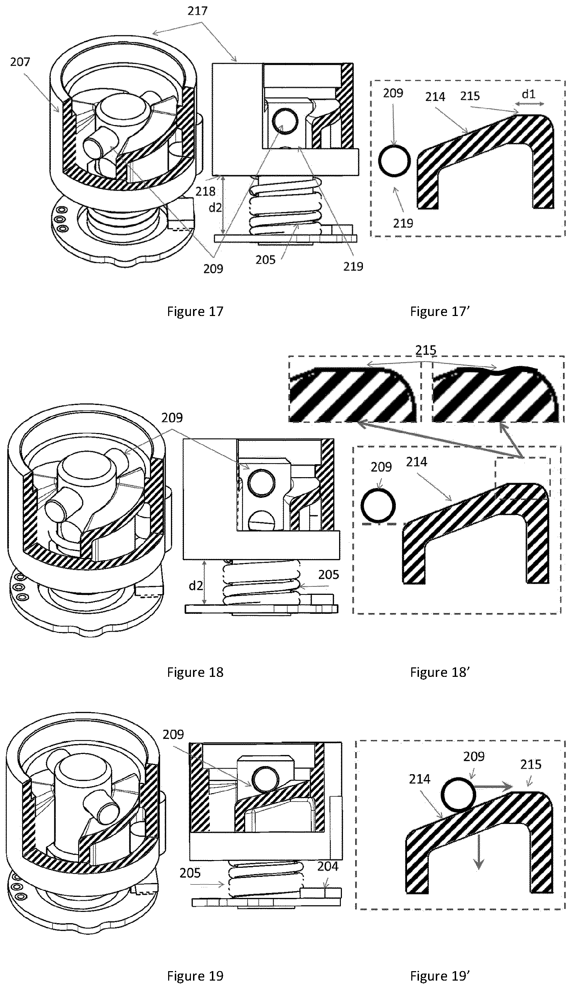

Figure 17', 18', 19', 20' et 21' illustrent la coopération entre la rampe et le moyen d'appui entrainé par le moteur (non représenté)

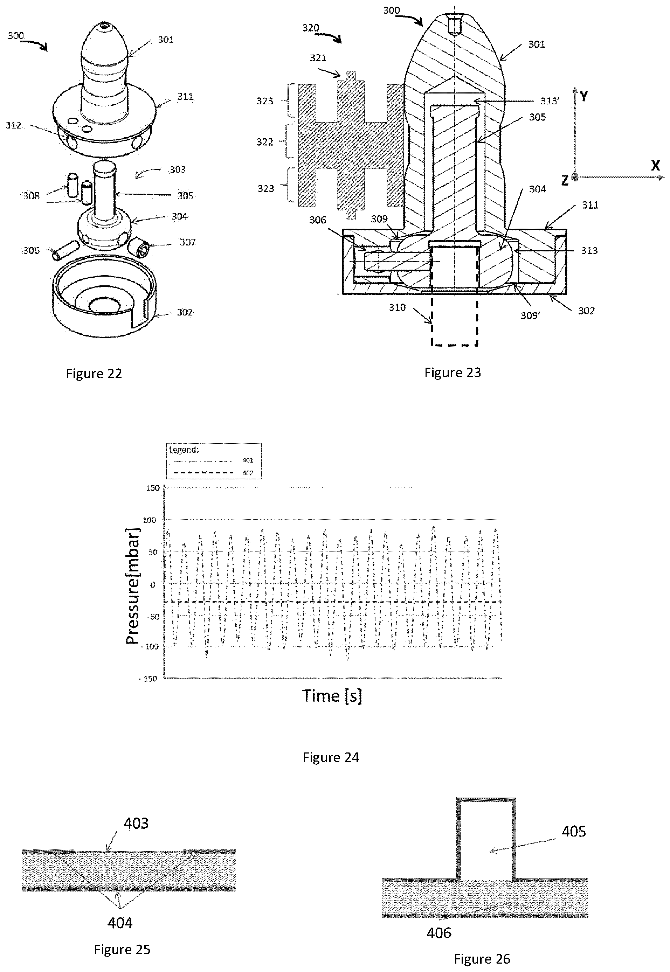

Figure 22 montre une vue éclaté du dispositif d'entrainement de la pompe péristaltique selon l'invention
Figure 23 montre une vue en coupe du dispositif d'entrainement de la pompe péristaltique selon l'invention
Figure 24 montre sur un graphique des pics de pression causés par une pompe
Figure 25 et 26 montrent deux conceptions différentes du moyen d'amortissement

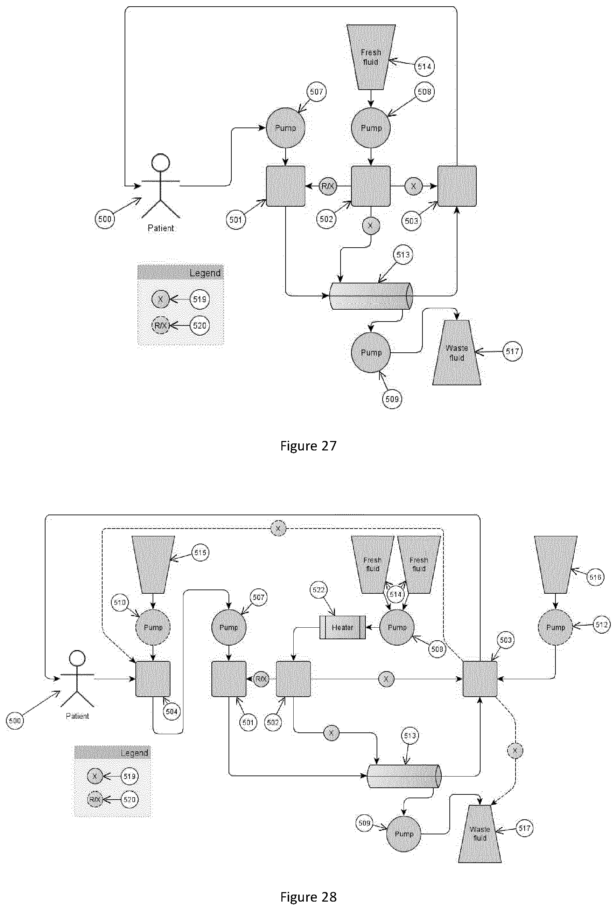

Figure 27 schématise un mode de réalisation minimale du système de traitement du sang

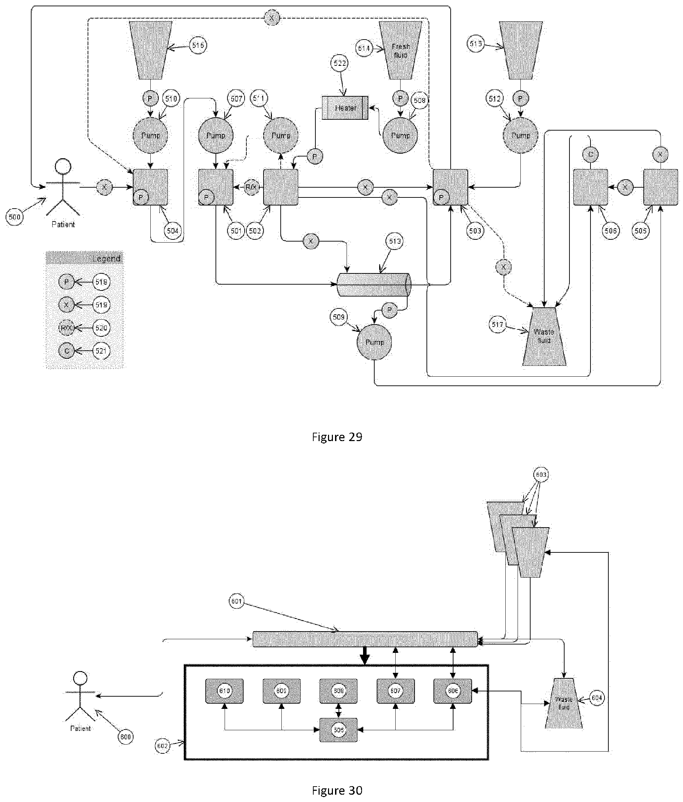

Figure 28 et 29 schématisent des modes réalisation plus complexe du système de traitement du sang

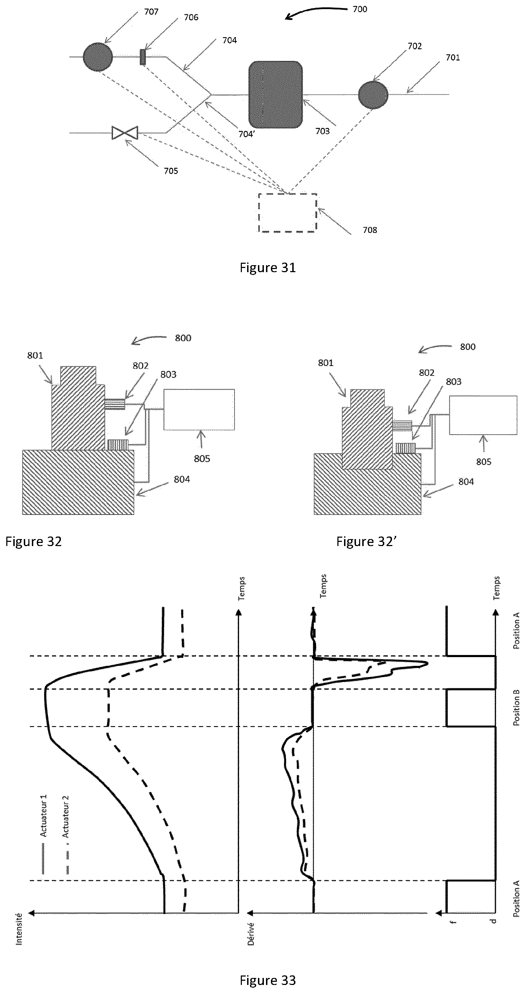

Figure 31 schématise l'utilisation de la pompe additionnelle pour la répartition du fluide
Figure 32 et 32' illustre l'utilisation d'un actionneur linéaire
Figure 33 représente 3 graphiques utilisés par un système de commande d'actionneur

### Références numériques utilisées dans les figures

- 1: Patient
- 2: Cassette
- 2': Cassette comprenant les pompes
- 3: Moyen de filtration du sang
- 4: Moyen de chauffage
- 5: Tuyau de sortie provenant du patient
- 6: Tuyau d'entré en direction du patient
- 7: Elément de sécurité
- 8: Entrée du sang dans le filtre
- 9: Entrée du dialysat dans le filtre
- 10: Sortie du filtrat provenant du filtre
- 11: Sortie du sang provenant du filtre
- 12: Membrane du filtre
- 13: Moyen d'ajustement de flux
- 14: Système de traitement du sang
- 15: Capteur volumique du troisième chemin fluidique
- 16: Capteur volumique de référence
- 17: Capteur volumique du premier chemin fluidique
- 100: Système de distribution de fluide
- 101: Zone de mesure
- 102: Canal
- 103: Chemin fluidique
- 104: Membrane
- 105: Corps rigide de la cassette
- 106: Ouverture
- 107: Capteur de pression
- 108: Filtre hydrophobe
- 200: Actionneur liénaire
- 201: Moteur DC avec réducteur
- 202: Enveloppe rigide
- 203: Rainure dans l'enveloppe rigide
- 204: Capteur
- 205: Moyen de compression
- 206: Aimant
- 207: Piston
- 208: Axe du moteur
- 209: Axe transversale
- 210: Elément de liaison du piston au téton
- 211: Téton
- 212: Valve
- 213: Siège de valve
- 214: Rampe
- 215: Seuil au sommet de la rampe
- 216: Moyens de guidage
- 217: Extrémité distale du piston
- 218: Extrémité proximale du piston
- 219: Passage
- 220: Direction 1
- 221: Direction 2
- 300: Dispositif d'entrainement
- 301: Axe flottant
- 302: Couvercle
- 303: Moyen d'entrainement
- 304: Corps du moyen d'entrainement
- 305: Axe longitudinal
- 306: Axe perpendiculaire
- 307: Vis defixation
- 308: Eléments durs
- 309 et 309': parois intérieur de la cavité
- 310: Rotor / moteur
- 311: Base
- 312: Elément de coopération
- 313: Cavité
- 313': Deuxième cavité
- 320: Galet
- 321: Axe du galet
- 322: Partie rigide
- 323: Partie flexible
- 401: Courbe de pression
- 402: Courbe de la pression moyenne
- 403: Membrane flexible
- 404: Paroi du chemin fluidique
- 405: Fluide compressible (exemple gaz)
- 406: Fluide
- 500: Patient
- 501: 1ère chambre
- 502: 2ème chambre
- 503: 3ème chambre
- 504: 4ème chambre
- 505: 5ème chambre
- 506: 6ème chambre
- 507: 1ère pompe
- 508: 2ème pompe
- 509: 3ème pompe
- 510: 4ème pompe (optionelle)
- 511: 5ème pompe (optionelle)
- 512: 6ème pompe (optionelle)
- 513: Filtre
- 514: 1er moyens d'alimentation en fluide
- 515: 2ème moyens d'alimentation en fluide
- 516: 3ème moyens d'alimentation en fluide
- 517: Moyens de récupération de fluide
- 518: Capteur de pression
- 519: Moyens d'obturation (par exemple valve)
- 520: Moyens de restriction du flux ou d'obturation
- 521: Capteur
- 522: Moyens de chauffage
- 600: Patient
- 601: Casette
- 602: Appareil de dialyse / boitier
- 603: Moyens d'alimentation en fluide
- 604: Moyens de récupération de fluide
- 605: Processeur
- 606: Capteurs
- 607: Actionneurs (Pompe, valve,...)
- 608: Ecran
- 609: Moyen d'acquisition et/ou autre, par exemple moyens d'alimentation en énergie
- 610: Mémoire
- 700: Système de distribution de fluide
- 701: Chemin fluidique principale
- 702: Pompe de précision
- 703: Poche souple (par exemple poche de chauffage souple)
- 704 et 704': Chemin fluidique secondaire
- 705: Valve
- 706: Capteur de pression
- 707: Pompe additionnelle
- 708: Processeur
- 800: Système de commande
- 801: Partie mobile de l'actionneur
- 802: Elément 1 du capteur
- 803: Elément 2 du capteur
- 804: Partie fixe de l'actionneur
- 805: Elément de commande (processeur et/ou autre élément)
- C1: Première chambre de distribution
- C1.1: Chambre de raccordement supplémentaire
- C1.2: Chambre de distribution supplémentaire
- C2: Première chambre de raccordement
- C3: Deuxième chambre de raccordement
- C4: Troisième chambre de raccordement
- C5: Quatrième chambre de raccordement
- C6: Cinquième chambre de raccordement
- C7: Deuxième chambre de distribution
- C8: Sixième chambre de raccordement
- C9: septième chambre de raccordement
- F1: Premier moyen d'alimentation en liquide
- F2: Deuxième moyen d'alimentation en liquide
- F3: Troisième moyen d'alimentation en liquide
- F4: Moyen de récupération du filtrat
- F5: Moyen de récupération du sang ou échantillonnage
- FI1: Fluide 1
- FI2: Fluide 2
- FI3: Fluide 3
- P1: Pompe du deuxième chemin fluidique (Sang)
- P2: Pompe principale du troisième chemin fluidique (Dialysat ou substitution)
- P2': Pompe additionnelle du troisième chemin fluidique
- P3: Pompe du premier chemin fluidique (Filtrat)
- P4: Pompe du deuxième moyen d'alimentation en liquide
- P5: Pompe du troisième moyen d'alimentation en liquide
- V1: Canal d'entrée du sang dans la troisième chambre de raccordement
- V1': Canal d'entrée du deuxième moyen d'alimentation en liquide dans la troisième chambre de raccordement
- V1": Canal d'entrée du sang dans la troisième chambre de raccordement provenant de la deuxième chambre de raccordement
- V2: Canal de sortie du sang dans la troisième chambre de raccordement
- V3: Canal d'entrée du sang dans la première chambre de raccordement
- V4: Canal de sortie du sang dans la première chambre de raccordement
- V5: Canal d'entrée du dialysat ou produit de substitution (pré dilution) dans la première chambre de raccordement
- V5': Canal de sortie du dialysat ou produit de substitution (pré dilution) de la chambre de distribution supplémentaire (C1.2) vers la première chambre de raccordement
- V6: Canal de sortie du dialysat ou produit de substitution (pré dilution) de la première chambre de distribution vers la première chambre de raccordement
- V6': Canal de sortie du dialysat ou produit de substitution de la première chambre de distribution vers la chambre de distribution supplémentaire (C1.2)
- V7: Canal de sortie du dialysat ou produit de substitution de la première chambre de distribution vers le filtre
- V7': Canal de sortie du dialysat ou produit de substitution de la première chambre de distribution vers la chambre de raccordement supplémentaire (C1.1)
- V7": Canal de sortie du dialysat ou produit de substitution de la chambre de raccordement supplémentaire (C1.1) vers le filtre
- V7‴: Canal de sortie du dialysat ou produit de substitution de la chambre de distribution supplémentaire (C1.2) vers la chambre de raccordement supplémentaire (C1.1)
- V8: Canal de sortie du dialysat ou produit de substitution (post dilution) de la première chambre de distribution vers la deuxième chambre de raccordement
- V9: Canal d'entrée du dialysat ou produit de substitution dans la première chambre de distribution
- V10: Canal de sortie du sang vers le patient
- V10': Canal de sortie du sang vers troisième chambre de raccordement
- V10": Canal de sortie du sang vers un moyen de récupération
- V11: Canal d'entrée du sang dans la deuxième chambre de raccordement en provenance du filtre
- V12: Canal d'entré du dialysat ou produit de substitution (post dilution) en provenance de la première chambre de distribution
- V12': Canal d'entrée du troisième moyen d'alimentation en liquide dans la deuxième chambre de raccordement
- V13: Canal de sortie du dialysat ou produit de substitution
- V14 et V14': Canal d'entrée du dialysat ou produit de substitution du premier moyen d'alimentation
- V15: Canal de sortie du filtrat de la deuxième chambre de distribution en direction de la sixième chambre de raccordement
- V16: Canal de sortie du filtrat de la deuxième chambre de distribution en direction de la septième chambre de raccordement
- V17: Canal d'entrée dans la septième chambre de raccordement en provenance du capteur volumique de référence
- V18: Canal de sortie du volume à mesurer
- V19: Canal d'entrée dans la sixième chambre de raccordement du volume à mesurer (dialysat)
- V20: Canal d'entrée dans la sixième chambre de raccordement du volume à mesurer (filtrat)

### Description détaillée de la divulgation

Dans le présent document, la description détaillée de la divulgation comporte des modes de réalisation de dispositifs, de systèmes et de méthodes présentés à titre d'illustration. Il est bien entendu que d'autres modes de réalisation sont envisageables et peuvent être apportées sans s'écarter de la portée de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés dans le présent document dans un sens large et signifie de façon générale « inclut, mais sans s'y limiter.

Le terme "ou" est généralement employé dans un sens large comprenant "et/ou" à moins que le contexte indique clairement le contraire.

Dans la présente divulgation, un canal peut être définit comme étant un conduit d'écoulement de forme creuse et allongée, permettant le passage d'un liquide et/ou d'un gaz d'un endroit à un autre. Il peut prendre la forme d'un tuyau flexible, d'une tubulure ou d'une cavité à l'intérieur d'une cassette. Certains canaux disposent de valves qui peuvent être actionnées préférentiellement grâce à un actionneur linéaire commandé par un contrôleur pour fermer ou ouvrir le canal. Sans actionnement, lesdites valves sont préférentiellement fermées. Une chambre peut être une cavité ou un canal ayant plusieurs entrées et/ou sorties ou prendre la forme d'une simple intersection de deux canaux. Chaque chambre dispose d'une entrée appelée canal d'entrée et d'une sortie appelé canal de sortie.

### Principe de la divulgation concernant un système de traitement du sang

Le mode de réalisation schématisé dans la figure 27 est un mode de réalisation simplifié. Le système de traitement comprend trois pompes (507, 508, 509), trois chemins fluidiques et un moyen de filtration ou filtre (513). Le premier chemin fluidique part du moyen de filtrage (513), jusqu'au réservoir (517) appelé également moyen de récupération de fluide. Le fluide qui s'écoule à travers le premier chemin fluidique est appelé le filtrat. Le deuxième chemin fluidique part du patient passe à travers le filtre (513) et retourne au patient (500). Le fluide qui s'écoule à travers le deuxième chemin fluidique est le sang du patient. Le troisième chemin fluidique est alimenté par un fluide appelé généralement dialysat (mais l'invention ne se limite pas à ce fluide) qui s'écoule du premier moyen d'alimentation en fluide appelé également réservoir de dialysat (514). Le dialysat s'écoule dans un premier temps dans le troisième chemin fluidique pour ensuite s'écouler dans un ou plusieurs chemin fluidique secondaire (sous forme dilué ou transformé). Le dialysat (ou autre fluide du troisième chemin fluidique) peut :
- se mélanger au sang :
   ∘ avant le filtre (513) pour effectuer une pré dilution, et/ou,
- après le filtre (513) pour effectuer une post-dilution, et/ou
- alimenter le filtre (513).

Pour permettre ces différentes solutions et donc effectuer n'importe quelle technique de traitement par dialyse, le système a besoin de trois chambres (501, 502, 503). La deuxième chambre (502), appelé également chambre de distribution, permet d'aiguiller le dialysat vers le filtre (513), la première chambre (501) pour effectuer une pré dilution et/ou la deuxième chambre (502) pour effectuer une post dilution. Les première et troisième chambres (501, 503) peuvent aussi être appelées chambre de raccordement. Les première et troisième chambres (501, 503) permettent de mélanger au sang du dialysat, autrement dit, Les première et troisième chambres (501, 503) permettent au fluide du troisième chemin fluidique de s'écouler dans le deuxième chemin fluidique. Les canaux qui relie les chambres entre-elle ou au filtre peuvent comprendre des valves (519) et/ou un moyen de restriction du flux ou d'obturation (520).

La figure 28 schématise un système plus complexe en rajoutant de nouveaux éléments optionnels. Il peut s'agir par exemple :
- d'un nouveau moyen d'alimentation (515) pouvant contenir par exemple un anticoagulant. Ce fluide peut être contenu dans un réservoir (515) et propulsé par une pompe (510) ou par l'apesanteur. Ce moyen d'alimentation peut être une seringue, et/ou
- d'un autre moyen d'alimentation (516) pouvant contenir par exemple un agent inhibant l'anticoagulant. Ce fluide peut être contenu dans un réservoir (516) et propulsé par une pompe (512) ou par l'apesanteur. Ce moyen d'alimentation peut être une seringue, et/ou
- Un nouveau canal permettant de relié la troisième chambre (503) (celle normalement utilisé pour la post-dilution) - ou à une autre chambre (localisée en aval de la troisième chambre) - à la première chambre (501) (celle normalement utilisé pour la pré dilution) - ou à une autre chambre (504) (localisée en amont de la première chambre) -, ce nouveau canal comprend de préférence une valve commandé par un contrôleur. Il permet au fluide contenu dans le deuxième chemin fluidique de circuler en boucle afin de ne pas stagner dans les canaux, et/ou
- Un autre canal permettant de relier la troisième chambre (503) au moyen de récupération de fluide (517). Ce canal comprend de préférence une valve. Il peut permettre par exemple le priming du système ou de jeter une partie du fluide s'écoulant dans le deuxième chemin fluidique, et/ou
- Un moyen de chauffage (522)

Un autre mode de réalisation est divulgué par la figure 29. Ce mode réalisation peut comprendre au moins un capteur de pression (518) - localisé dans ou proche des chambres (504, 501, 502, 503), du filtre (513) et/ou des moyen d'alimentation (515, 516, 514) - et/ou une pompe additionnelle (511) (à la place du restricteur de flux (520)) dans le troisième chemin fluidique entre la chambre de distribution (502) et la chambre de raccordement (501). Le système peut comprendre en outre système d'étalonnage pouvant comprendre un capteur commun au premier et troisième chemin fluidique. Ce système d'étalonnage comprend deux canaux supplémentaires qui permettent aux fluides contenus dans le premier et troisième chemin fluidique de s'écouler vers une sixième chambre (506). Cette sixième chambre (506) comprend dans ou relié à elle un capteur permettant l'étalonnage d'éléments (capteurs et/ou pompe) du premier et troisième chambre afin qu'il soit étalonné de façon identique.

La figure 30 divulgue un système de traitement tel que décrit précédemment. Ce système comprend en outre une cassette (601) permettant d'assurer les fonctions de distribution de fluides. Cette cassette (601) est reliée à des réservoirs (603, 604) et coopère avec un dispositif (602). Le dispositif (602) appelé appareil de dialyse peut être réutilisable alors que la cassette (601) peut être jetable. Le dispositif (602) peut comprendre un processeur (605), au moins un capteur (606) adapté pour coopérer avec la cassette (601), au moins un actionneur (607) (par exemple pompe ou moyen de commande) adapté pour coopérer avec la cassette (601), un écran (608), au moins un moyen d'acquisition et/ou autre comme une batterie (609) et/ou une mémoire (610).

### Modes réalisation de cassettes selon le principe de fonctionnement décrit précédemment

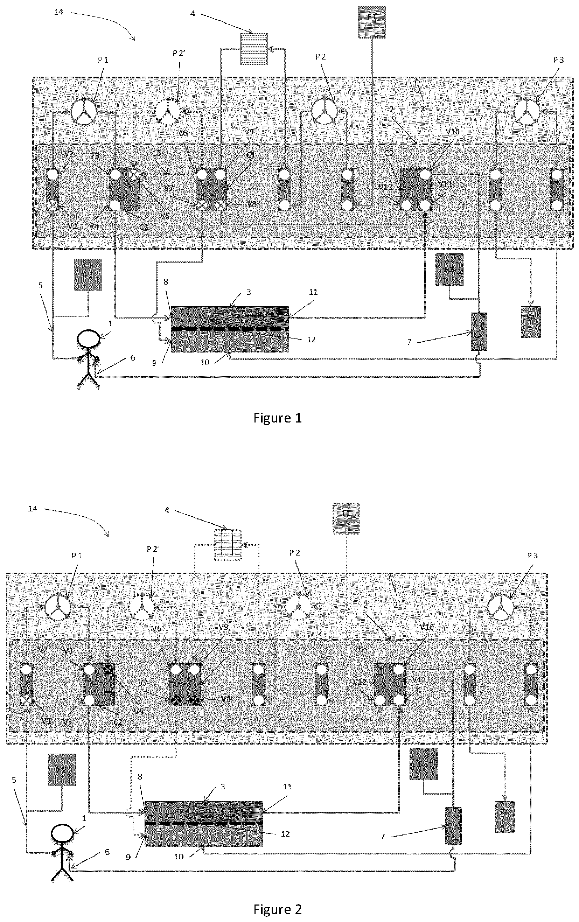

Selon la figure 1 la divulgation divulgue un système (14) qui permet d'effectuer un traitement du sang d'un patient et qui comprend un moyen de filtration du sang (3), au moins un moyen d'alimentation en liquide (F1), deux tuyaux patient, un tuyau de sortie (5) prélevant le sang à traiter et un tuyau d'entré (6) réinjectant le sang traité audit patient, un moyen de récupération du filtrat (F4), au moins trois pompes à fluide (P1, P2, P3), une cassette (2, 2') composée de canaux et de valves de manière à diriger les fluides. Ladite casette (2, 2') comprend aux moins une chambre de distribution (C1, C1.2, C7) comprenant un seul canal d'entrée et au moins deux canaux de sortie. Ledit système de traitement (14) comprend un contrôleur qui commande l'ouverture et la fermeture desdites valves en fonction du traitement souhaité.

Ledit système de traitement (14) comprenant en outre un premier chemin fluidique, reliant ledit moyen de filtration du sang (3) au moyen de récupération du filtrat (F4), composé d'une série de canaux et d'une pompe dédiée (P3) ; un deuxième chemin fluidique dédié à la circulation du sang, comprenant une série de canaux, ledit moyen de filtration du sang (3), lesdits tuyaux patient (5, 6) et une pompe dédiée (P1) ; un troisième chemin fluidique composé d'un moyen d'alimentation en liquide (F1), d'au moins une pompe dédiée (P2), d'une série de canaux, d'un moyen de chauffage (4) et d'au moins une chambre de distribution (C1, C1.2).

Avantageusement, ladite chambre de distribution (C1) comprend au moins trois canaux de sortie (distincts) raccordés directement ou indirectement :
- au deuxième chemin fluidique en amont dudit moyen de filtration du sang (3),
- au deuxième chemin fluidique en aval dudit moyen de filtration du sang (3),
- audit moyen de filtration du sang (3)

Ladite cassette peut comprendre en outre au moins un moyen de régulation de flux conçu pour maîtriser la quantité de liquide du troisième chemin fluidique qui s'écoule dans au moins un desdits canaux de sortie de ladite chambre de distribution (C1). De plus, ledit au moins un moyen de régulation de flux est commandé par ledit contrôleur qui peut comprendre le processeur (605).

Ladite cassette (2') peut contenir également les pompes et/ou d'autres éléments.

Le système de traitement comprend en outre, dans le troisième chemin fluidique, un moyen d'ajustement de flux (13, P2') localisé entre ladite chambre de distribution (C1) et ladite première chambre de raccordement (C2).

Dans un mode de réalisation, le troisième chemin fluidique comporte une pompe additionnelle (P2') dans le troisième chemin fluidique, localisé entre la chambre de distribution (C1) et ladite première chambre de raccordement (C2). La dite pompe additionnelle (P2') joue le rôle total ou partiel de moyen d'ajustement de flux. Le moyen d'ajustement de flux a pour objectif de contrôler l'écoulement du fluide passant de la chambre de distribution (C1) à la chambre de raccordement (C2). Le moyen d'ajustement permet ainsi de répartir la quantité de fluide dans la chambre de raccordement (C2) et au moins une autre chambre de raccordement ou le moyen de filtration du sang (3).

Dans un mode de réalisation, un ou plusieurs moyens d'ajustement de flux (appelé également restricteur de flux) peuvent être placé entre n'importe quelle chambre ou élément (par exemple moyen de filtration du sang (3)). Un moyen d'ajustement de flux (13) peut être une pompe, une valve proportionnelle et/ou un ensemble de canaux à valve dédiée et de diamètre différents,... Une valve supplémentaire de sécurité peut être ajoutée en amont ou aval du moyen d'ajustement de flux (13 - 520). Un moyen d'ajustement de flux permet un écoulement de 0% à 100% du fluide à un moment ou durant une durée donné à travers ledit moyen d'écoulement. Autrement dit, l'écoulement d'un fluide provenant d'au moins un moyen d'alimentation en liquide peut être réparti dans les différents canaux selon les besoins du traitement.

Dans un mode de réalisation, le système de traitement (14) comprend un deuxième moyen d'alimentation (F2 - 515) en liquide localisé sur le tuyau de sortie du patient (5) ou dans la cassette (2, 2' - 601). Ledit deuxième moyen d'alimentation peut contenir un anticoagulant tel que du citrate, de l'héparine, du danaparoïde sodique ou autre.

Dans un mode de réalisation, ledit système (14) comprend un troisième moyen d'alimentation en liquide (F3 - 516) localisé sur le tuyau d'entré (6) du patient ou dans la cassette (2, 2' - 601). Ledit moyen d'alimentation en liquide pouvant contenir du calcium ou un agent inhibant l'anticoagulant.

Sur le tuyau d'entrée patient (6) et/ou dans la cassette (2, 2' - 601), le système comprend au moins un élément de sécurité (7) permettant de détecter les bulles d'air dans le deuxième chemin fluidique et/ou stopper la circulation du sang et/ou un moyen de capturer lesdites bulles d'air.

### Localisation du moyen de chauffage et/ou utilisation de deux pompes dans le troisième chemin fluidique :

Selon le principe de fonctionnement divulgué par la figure 31, pour être efficace, le système de distribution de fluide (que ce soit pour une cassette tel que décrite dans le présent document ou pour un autre système de distribution) peut comprendre une pompe de précision (702), un moyen d'alimentation en fluide, une poche souple (703) et une pompe additionnelle (707) de répartition. Le système comprend en outre un chemin fluidique principal (701) qui se divise en au moins deux chemins fluidiques secondaires (704, 704') et distincts. La pompe de précision (702) et la poche souple (703) sont positionnées dans le chemin fluidique principal (701), la poche souple étant positionnée en aval de ladite pompe. Ainsi tout le fluide pompé est connu avec précision et peut être au moins partiellement stocké au moins temporairement dans la poche souple. La pompe additionnelle (707) est positionnée dans un des chemins fluidiques secondaires (704). Préférentiellement, l'autre chemin fluidique secondaire (704') comprend une valve (705). Dans un mode de réalisation, au moins un chemin fluidique comprend un capteur de pression (706) positionné en aval de la pompe de précision (702). Préférentiellement, ledit capteur de pression (706) est positionné dans le chemin fluidique secondaire (704) qui comprend la pompe additionnelle (707) et en amont de la pompe additionnelle. Préférentiellement la poche souple (703) est un moyen de chauffage.

Etant donné que certaines techniques d'épuration extra rénale continue nécessitent de chauffer le dialysat et/ou liquide de substitution durant son injection, ledit moyen de chauffage (4) peut être localisé à différents endroit du troisième chemin fluidique. Dans un mode de réalisation, la cassette dispose d'un moyen de chauffage (4) à l'intérieur de la chambre de distribution (C1) ou en amont de cette dernière.

Dans un mode de réalisation selon le principe décris précédemment, le moyen de chauffage (4) est une poche souple, localisée entre de la pompe principale (P2) dudit troisième chemin fluidique et ladite chambre de distribution (C1) qui permet de créer une pression positive constante dans ladite poche (4). La poche de chauffage est alimentée en continu par la pompe (P2) ce qui permet entre autre de garantir une bonne maîtrise du réchauffement du liquide du troisième chemin fluidique. Ladite poche (4) est ensuite directement reliée au canal d'entrée (V9) de la chambre de distribution (C1).

De par cette configuration, tout le liquide injecté passe par une seule pompe (P2). La pompe de pré dilution (P2') (appelé également pompe additionnelle) ne fait que répartir le liquide avant et/ou après le filtre. Ainsi, une seule pompe de précision est nécessaire. La pompe (P2) est la pompe de précision et permet de connaître la quantité de fluide pompé. La pompe additionnelle permet simplement la répartition entre la pré dilution (avant le filtre) et la post dilution (après le filtre). L'utilisation des pompes s'effectuent comme-suit :
- Si seulement de la post dilution est programmée : la pompe de pré dilution (P2') est stoppée et tout le liquide sera injecté après le filtre (3). La valve de post dilution (V8) est ouverte. Préférentiellement, la valve de pré dilution (V5) est fermée.
- Si seulement de la pré dilution est programmée : la pompe de post dilution (P2) délivre le volume de substitution. La valve de post dilution (V8) empêche le passage du liquide après le filtre. La pompe de pré dilution (P2') permet également de réguler le fluide pour éviter que la pression dans le moyen de chauffage (4) ne devienne négative.
- Si de la pré et post dilution sont programmées : la pompe principale (P2) délivre tout le volume de substitution requit (pré et post). La valve de post dilution (V8) est ouverte. La pompe pré dilution (P2') ponctionne une partie du liquide pour l'injecter avant le filtre (3). Si une erreur de précision existe dans la répartition pré et post injection, sa gravité est limitée car le volume est de toute façon injecté au patient.

### Utilisation de la cassette en fonction des différents traitements :

- Ultrafiltration continue lente (SCUF) :
   La figure 2 représente l'utilisation de la cassette appliquant un traitement d'ultrafiltration continue lente. Cette technique est utilisée pour éliminer une surcharge liquidienne grâce au principe de convection.
   Ainsi, le contrôleur ouvre uniquement la valve V1 du deuxième chemin fluidique et ferme les valves V5, V7 et V8 du troisième chemin fluidique. Les pompes P1 et P3 fonctionnent alors que P2 et P2' ne fonctionnent pas.
- Hémofiltration veino-veineuse continue (CVVH) :

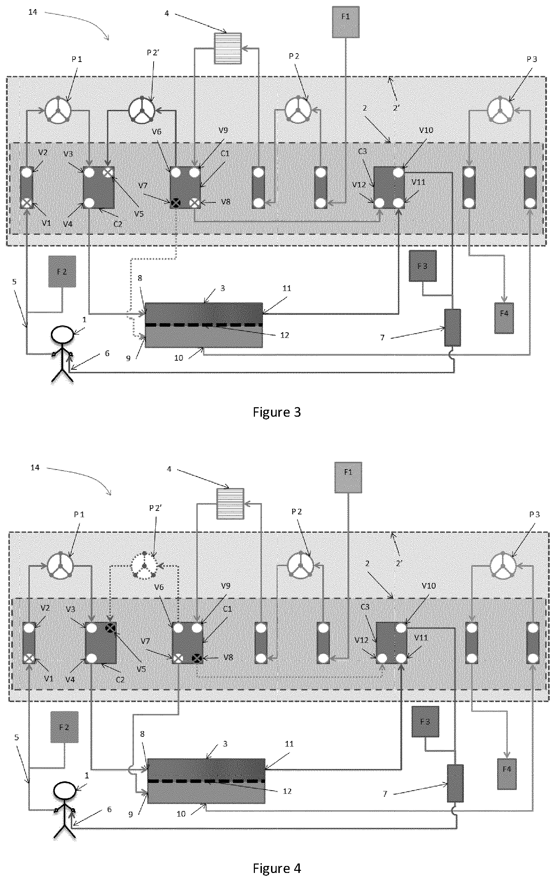

   La figure 3 représente l'utilisation de la cassette appliquant un traitement d'hémofiltration veino-veineuse continue. Cette technique est utilisée pour obtenir le retrait de substances dissoutes grâce au principe de convection. Une solution de substitution est injectée dans le circuit avant (la prédilution) et/ou après (la post - dilution) le moyen de filtration (3).
   Ainsi, le contrôleur ouvre les valves V1 du deuxième chemin fluidique et V5 et/ou V8 du troisième chemin fluidique et la valve V7 est fermée. Les pompes P1, P2 (optionnellement P2') et P3 fonctionnent.
- Hémodialyse veino-veineuse continue (CVVHD) :
   La figure 4 représente l'utilisation de la cassette appliquant un traitement d'hémodialyse veino-veineuse continue. Cette technique est utilisée pour obtenir le retrait de substances dissoutes (petites molécules: urée, créatine, K,...) et obtenir un équilibre hydrique par principe de diffusion. Le dialysat est injectée dans le moyen de filtration (3).
   Ainsi, le contrôleur ouvre les valves V1 du deuxième chemin fluidique et V7 du troisième chemin fluidique et les valves V5 et V8 restent fermées. Les pompes P1, P2 et P3 fonctionnent.
- Hémodiafiltration veino-veineuse continue (CWHDF) :

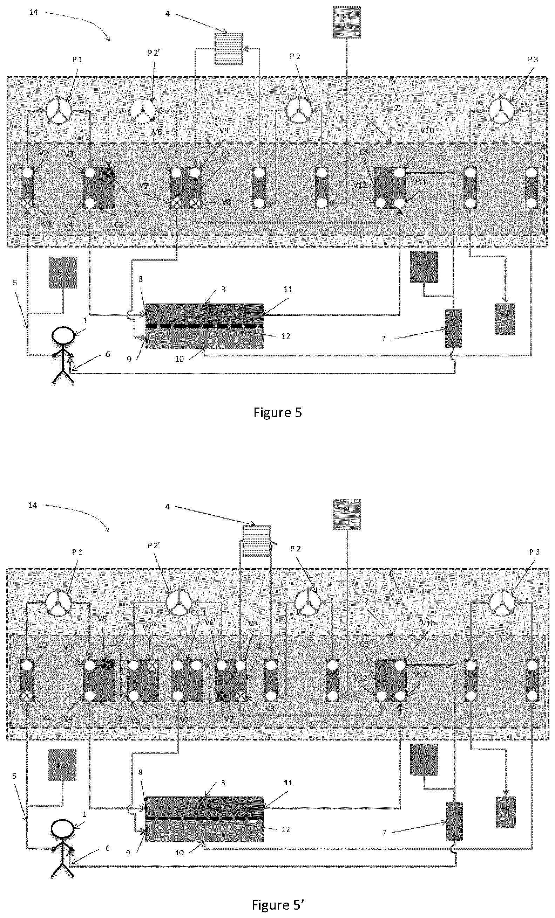

   La figure 5 représente l'utilisation de la cassette appliquant un traitement d'hémodiafiltration veino-veineuse continue. Cette technique est utilisée pour obtenir le retrait de substances dissoutes (petites ou moyennes molécules) grâce aux principes de diffusion et de convection. Le dialysat et/ou une solution de substitution sont injectés dans le moyen de filtration (3) et dans le sang après le moyen de filtration (3).
   Ainsi, le contrôleur ouvre la valve V1 du deuxième chemin fluidique ainsi que les valves V7 et V8 du troisième chemin fluidique. La valve V5 reste fermée. Les pompes P1, P2 et P3 fonctionnent. Dans ce mode réalisation, les canaux de sortie V7 et V8 sont des valves proportionnelles ou autre moyen de contrôler le flux qui passe à travers ces valves.
   Dans un autre mode de réalisation exposé en figure 5', la cassette comprend une chambre de distribution supplémentaire (C1.2) et une chambre de raccordement supplémentaire (C1.1). Ladite chambre de distribution supplémentaire (C1.2) est directement alimentée par la pompe additionnelle (P2'). Ladite chambre de distribution supplémentaire (C1.2) permet de distribuer le dialysat ou produit de substitution soit à la première chambre de raccordement (C2) pour la pré dilution soit à ladite chambre de raccordement supplémentaire (C1.1). Ladite chambre de raccordement supplémentaire (C1.1) est également alimentée en dialysat ou produit de substitution par la première chambre de distribution (C1) grâce au canal de sortie à valve dédiée (V7'). Ladite chambre de raccordement supplémentaire (C1.1) dispose également d'un canal de sortie (V7") qui se relie au filtre (3). L'intérêt d'une telle disposition est d'augmenter en précision les quantités injectées dans le filtre et dans la deuxième chambre de raccordement pour la post dilution ou dans le filtre et dans la première chambre de raccordement pour la pré dilution. Ainsi, la pompe additionnelle (P2') permet de distribuer avec précision les quantités de fluide à répartir. Dans ce mode de réalisation, pour un traitement d'hémodiafiltration veino-veineuse continue, les valves V1, V8 et V7" sont ouvertes.
- Echanges plasmatiques (TPE) :

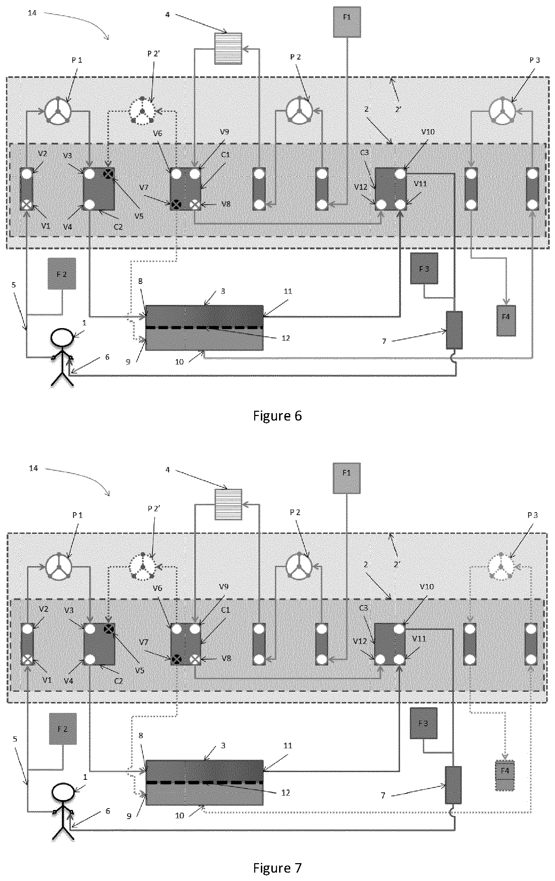

   La figure 6 représente l'utilisation de la cassette appliquant un traitement d'échanges plasmatiques. Cette technique permet des échanges plasmatiques par filtration membranaire. Une solution de substitution est injectée pour remplacer le plasma extrait.
   Ainsi, le contrôleur ouvre la valve V1 du deuxième chemin fluidique ainsi que la valve V8 du troisième chemin fluidique. Les valves V5 et V7 restent fermées. Les pompes P1, P2 et P3 fonctionnent. Préférentiellement F2 et F3 délivrent leur fluide dans le deuxième chemin fluidique.
- Hémoperfusion :
   La figure 7 représente l'utilisation de la cassette appliquant un traitement d'hémoperfusion. Cette technique est utilisée pour éliminer les substances toxiques du sang d'un patient, où le moyen de filtration contient une substance absorbante. Une solution de substitution est injectée pour remplacer le plasma extrait.
   Ainsi, le contrôleur ouvre la valve V1 du deuxième chemin fluidique ainsi que la valve V8 du troisième chemin fluidique. Les valves V5 et V7 restent fermées. Les pompes P1 et P2 fonctionnent, la pompe P3 ne fonctionne pas. Préférentiellement F2 et F3 délivrent leur fluide dans le deuxième chemin fluidique.

### Système disposant de plusieurs moyens d'alimentation en liquide

Lorsqu'un chemin fluidique (provenant par exemple d'un moyen d'alimentation en liquide supplémentaire) se relie à un autre chemin fluidique, la cassette comprend préférentiellement une chambre de raccordement permettant l'intersection desdits deux chemins fluidiques.

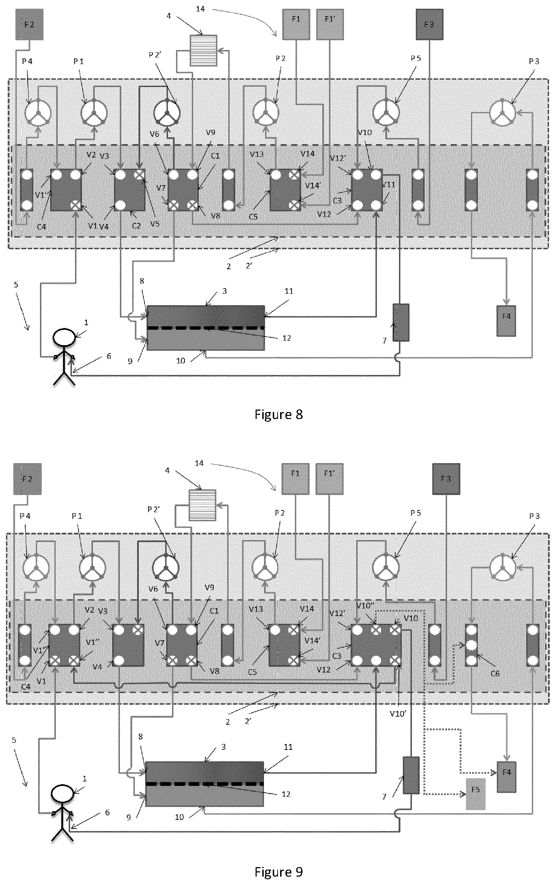

Dans un mode de réalisation décrit dans la figure 8, la cassette comprend :
- Une troisième chambre de raccordement (C4) comprenant une canal d'entrée (V1'), un canal d'entrée à valve dédiée (V1) et un canal de sortie (V2). Cette chambre de raccordement introduit un fluide contenu dans un deuxième moyen d'alimentation en liquide (F2) dans le chemin fluidique du sang (deuxième chemin fluidique), préférentiellement ledit deuxième moyen d'alimentation en liquide (F2) contient un agent anticoagulant, et/ou
- Une troisième canal d'entrée (V12') dans la deuxième chambre de raccordement (C3). Le troisième canal d'entrée (V12') permet d'injecter un fluide contenu dans un troisième moyen d'alimentation en liquide (F3) dans le chemin fluidique du sang (deuxième chemin fluidique), préférentiellement ledit troisième moyen d'alimentation en liquide (F3) contient un agent inhibant l'anticoagulant, et/ou
- Une quatrième chambre de raccordement (C5) comprenant au moins deux canaux d'entrée (V14, V14') à valve dédiée permettant d'avoir au moins deux fluides différents ou similaires dans le troisième chemin fluidique, par expemple du dialysat dans un sac et un produit de substituion dans un autre.

### Circulation sans interruption, vidange et priming du deuxième et/ou troisième chemin fluidique :

Dans un mode de réalisation décrit dans la figure 9, la deuxième chambre de raccordement (C3) comprend :
- Un canal d'entrée (V11) du deuxième chemin fluidique relié au moyen de filtration (3)
- Un canal d'entrée (V12) du troisième chemin fluidique relié à la chambre de distribution (C1)
- Trois canaux de sortie à valve dédiée (V10, V10', V10"), le premier étant relié au tuyaux d'entrée patient (6), le deuxième étant relié à un canal d'entrée de la deuxième chambre de raccordement (C4) et le troisième étant relié soit à un moyen de récupération (F5), soit directement ou indirectement au moyen de récupération du filtrat (C6).

Ce mode de réalisation permet par exemple :
- En cas de problème, le contrôleur peut fermer la valve V10 pour éviter par exemple d'injecter au patient une bulle d'air ou un autre élément pouvant mettre en danger la vie du patient. Dans ce cas, le sang restant dans la cassette et le filtre risque de coaguler. Il est donc impératif que le sang ne stagne pas dans la cassette, ni dans le filtre. Ainsi, P1 continue à fonctionner prélevant du sang depuis la deuxième chambre de raccordement afin de faire circuler en boucle sang entre la première, la deuxième chambre de raccordement et le filtre. V10' et/ou V1 "sont ouvert alors que V1, V10 et V10" sont fermé.
- d'effectuer des prélèvements du sang grâce à la valve V10",
- d'effectuer l'amorçage du traitement en faisant le vide d'air du système,
- de rincer le deuxième chemin fluidique avec le fluide du troisième chemin fluidique,
- d'éliminer tout ou partie du fluide contenu dans le deuxième et/ou du troisième chemin fluidique.

### Moyens et méthode de calibration des pompes et/ou des capteurs du premier et troisième chemin fluidique :

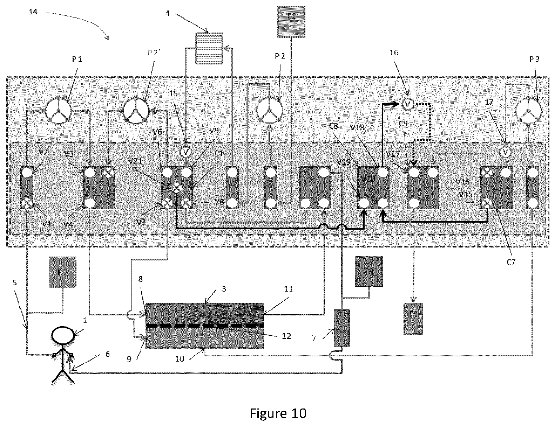

Certaines techniques d'épuration extra rénale continue nécessitent de connaître avec précision la quantité de volume injecté et retiré via respectivement le troisième et premier chemin fluidique. Le système de traitement comprend préférentiellement des pompes péristaltiques. Ce type de pompe peut connaître une certaine imprécision. Ainsi, selon la figure 10, pour connaitre avec précision la quantité de volume ajouté et retiré, le système de de distribution comprend au moins deux capteurs de volume permettant de mesurer les volumes des troisième et premier chemins fluidiques.

Le premier capteur (15) est compris dans le troisième chemin fluidique entre la chambre de distribution (C1) et la pompe principale (P2) et mesure le volume injecté provenant du premier moyen d'alimentation en liquide (F1, F1'). Préférentiellement, le capteur (15) est localisé après le moyen de chauffage (4). Le deuxième capteur (17) est placé dans le premier chemin fluidique en aval de la pompe et avant tout autre chambre. Ledit deuxième capteur (17) mesure le volume du filtrat retiré. Pour éviter tout risque de contamination, les deux capteurs sont localisés de préférence dans la cassette.

Dans un mode de réalisation préféré, le système de traitement comprend :
- un troisième capteur de volume (16) permettant de comparer les volumes mesurés par les deux précédents capteurs de volume (15, 17), ledit capteur est également appelé capteur de référence,
- un moyen de prélèvement des fluides provenant du premier et troisième chemin fluidique. Ledit moyen de prélèvement comprend une sixième chambre de raccordement (C8) disposant d'un canal de sortie directement relié audit troisième capteur (16) et de deux canaux d'entrée (V19, V20) raccordés respectivement à :
   ∘ un canal de sortie à valve dédiée (V21) localisé dans la première chambre de distribution,
   ∘ un canal de sortie à valve dédiée (V15) localisé dans la deuxième chambre de distribution
- optionnellement, une septième chambre de raccordement (C9) permettant aux capteurs de référence (16) de rejeter les liquides mesurés dans le moyen de récupération du filtrat (F4).

Pour éviter tout risque de contamination, ledit troisième capteur peut être localisé de préférence dans la cassette.

La méthode comprend les étapes suivantes :
- calibration du volume injecté :
   ∘ ouverture de la valve (V21) et fermeture des autres valves,
   ∘ actionnement de la pompe principale (P2) du troisième chemin fluidique,
   ∘ mesure du volume pompé par ladite pompe (P2) via ledit premier capteur (15) dudit troisième chemin fluidique,
   ∘ mesure dudit volume pompé via le capteur de référence (16),
   ∘ comparaison des deux mesures,
   ∘ calibration du premier capteur (15) et/ou de la pompe (P2).
- calibration du volume retiré :
   ∘ ouverture de la valve (V15) et fermeture des autres valves,
   ∘ actionnement de la pompe (P3) du filtrat du premier chemin fluidique,
   ∘ mesure du volume pompé par ladite pompe (P3) via ledit deuxième capteur (17) dudit premier chemin fluidique,
   ∘ mesure dudit volume pompé via le capteur de référence (16),
   ∘ comparaison des deux mesures,
   ∘ calibration du premier capteur (15) et/ou de la pompe (P2).

Ces étapes peuvent être effectuées lors du priming et/ou pendant le traitement.

Les premier et deuxième capteurs (15, 17) sont ajustés sur un capteur commun appelé capteur de référence (16) pour une précision relative optimum. Lesdits capteurs, même inexact, sont suffisamment efficaces puisqu'ils sont exacts en comparatif (relatif) par rapport au capteur de référence (16).

Ledit capteur de référence (16) peut-être une balance, une pompe volumétrique, un capteur de débit massique ou tout capteur permettant de mesurer ou déduire un volume.

Préférentiellement, lesdits premier et deuxième capteurs (15, 17) mesurent en continue les liquides passant respectivement dans le troisième et premier chemin fluidique. Grâce à la mesure en continu des volumes, la compensation d'une éventuelle dérive est possible.

### Capteur de pression déporté du chemin fluidique :

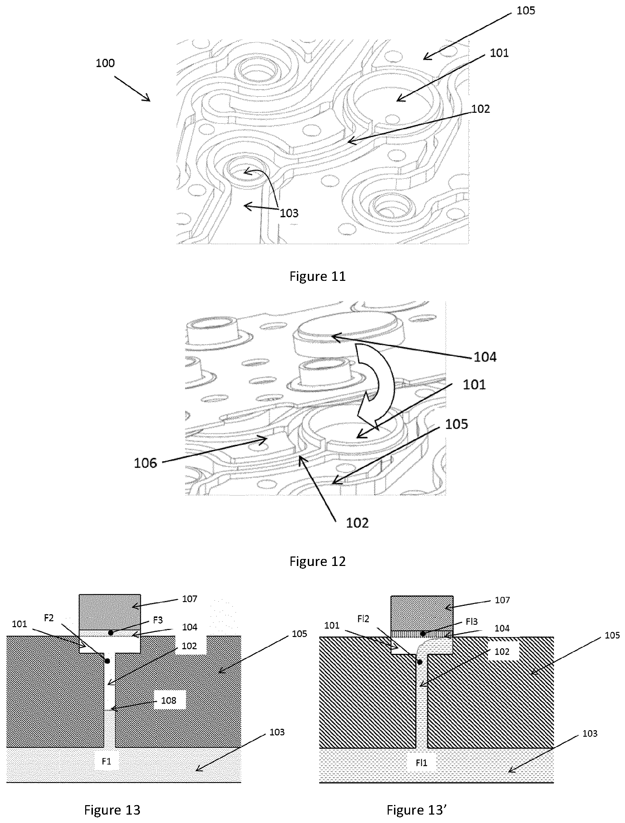

Selon les figures 11, 12 et 13, la présente invention divulgue un système de distribution de fluide (100) (préférentiellement une cassette tel que décrit précédemment) qui permet de prélever et/ou de délivrer un fluide FI1 du et/ou au patient et de mesurer la pression dudit fluide FI1. Le système comprend un corps rigide (105) composé d'au moins un chemin fluidique (103) à travers lequel ledit fluide FI1 s'écoule et d'au moins un canal (102). Ledit canal (102) est distinct du chemin fluidique (103) et permet de relier ledit chemin fluidique à une zone de mesure (101). Le système comprend en outre au moins une ouverture (106) recouverte par une membrane flexible (104) formant ladite zone de mesure. La membrane est façonnée pour recevoir un capteur de pression (107).

Un fluide FI2 différent du fluide FI1 est contenu dans ladite zone de mesure (101). Le fluide FI2 s'étend jusqu'à au moins en partie dans ledit canal (102). Ledit fluide FI2 permet de transmettre par contact la pression du fluide FI1 à ladite membrane (104). Ledit canal (102) est un restricteur de flux façonné de manière à ce que ledit fluide FI1 ne puisse entrer en contact avec ladite membrane. La longueur et/ou la forme dudit canal de transmission de pression (102) dépend de la capacité de dilatation dudit fluide FI2 et/ou de la gamme de pression à mesurer. Préférentiellement, le canal (102) comprend au moins une section suffisamment étroite pour retenir le fluide FI1 afin que ledit fluide ne pénètre pas dans ladite zone de mesure (101).

Dans un mode de réalisation, le canal (102) comprend un filtre hydrophobe (108) ou une membrane.

Dans un mode de réalisation, une interface membrane (104) / fluide (FI3) / cellule du capteur (107) est créée pour ne pas avoir de frictions de la membrane (104) sur ladite cellule qui pourraient crées des perturbations sur la mesure. Une interface liquide (FI1) / fluide (FI2) / membrane (104) est créé pour éviter que la membrane (104) ne soit mouillée par le liquide (FI1). La transmission des pressions de FI1 est assurée par les fluides FI2 et FI3, disposés de chaque côté de la membrane (104). FI2 et FI3 ont de préférence les mêmes propriétés physiques. Préférentiellement, FI2 et FI3 est de l'air. Ladite membrane (104) peut se déformer avec des contraintes équivalentes de chaque côté de ces faces et ce pour compenser les variations de volume d'air, piégé entre la membrane (104) et le capteur (107), dues à la température.

Dans un autre mode réalisation, le fluide FI1 est de nature aqueux alors que FI2 est de nature lipidique, FI3 pouvant être de nature lipidique ou aqueux.

Dans un autre mode de réalisation, la figure 13' divulgue un système de distribution de fluide (100) qui permet l'écoulement d'un fluide FI1 et de mesurer la pression dudit fluide FI1. Le système comprend un corps rigide (105) composé d'au moins un chemin fluidique (103) à travers lequel ledit fluide FI1 s'écoule et d'au moins un canal (102). Ledit canal (102) est distinct du chemin fluidique (103) mais communique afin que le fluide FI1 puisse s'écouler dans le canal (102). Ainsi, le canal (102) permet de relier ledit chemin fluidique à une zone de mesure (101). Le système comprend en outre au moins une ouverture (106) recouverte par une membrane flexible (104) formant ladite zone de mesure. Ladite ouverture (106) peut être de taille égale ou différente de la taille du canal (102). En outre, la membrane est façonnée pour recevoir un capteur de pression (107). Un fluide FI2 différent du fluide FI1 est contenu dans ladite zone de mesure (101). Le fluide FI2 est contenu au moins en partie dans la zone de mesure et/ou dans le canal (102).

Dans une mode de réalisation toujours illustré par la figure 13', la quantité et/ou le volume du fluide FI2 est constant ou peut diminuer aux cours du temps de manière à ce que le fluide FI1 progresse plus ou moins rapidement dans le canal (102) et/ou le zone de mesure (101).

Dans un mode de réalisation, la zone de mesure (101) et/ou le canal (102) contiennent au moins partiellement le fluide FI2 et le fluide FI1. Le fluide FI1 peut partiellement mouiller ou être en contact avec la membrane (104). La longueur et/ou la forme dudit canal de transmission de pression (102) dépend de la capacité de dilatation dudit fluide FI2 et/ou de la gamme de pression à mesurer.

Le canal (102) peut être façonné de manière à limiter et/ou freiner la progression du fluide FI1 par exemple durant l'utilisation dudit système. Le système de distribution de fluide (100) peut être adapté de manière à garantir à ce que la membrane (104) et/ou la zone de mesure (101) ne soient pas intégralement mouillées par ou en contact avec le fluide FI1 durant l'utilisation dudit système.

### Actionneur linéaire économique en énergie :

La divulgation divulgue un actionneur linéaire (200) utilisant un moteur (par exemple un moteur à courant continu (appelé également moteur DC) ou autre type de moteur connu par l'homme du métier) (201) couplé à des moyens interposés permettant de transformer la rotation de l'axe moteur en un mouvement linéaire. Préférentiellement, le moteur peut comprendre également un réducteur de couple.

En particulier, les moyens interposés comprennent :
- au moins une rampe (214) périphérique disposée à l'intérieur d'un piston (207),
- au moins un moyen d'appui (209) fixé directement ou indirectement au rotor (208) dudit moteur électrique(201), ledit moyen d'appui (209) étant façonné de manière à coopérer avec ladite rampe (214) périphérique,
- au moins un moyen de guidage (203, 216) permettant au piston (207) de guider le mouvement de translation.

Ladite rampe (214) comprend au moins un seuil, dont un seuil (215) est localisé au sommet de ladite rampe (214). Dans un mode réalisation, au moins un seuil peut être façonné de manière à coopérer avec le moyen d'appui. Par exemple, le seuil peut être parfaitement plat, horizontal par rapport au mouvement vertical du piston. Le seuil peut également présenter une forme spécifique pour assurer un bon maintien du moyen d'appui afin de garantir le maintien de la position, par exemple, le mode de réalisation de droite de la figure 18'. En outre, le seuil (215) localisé au sommet de ladite rampe (214) peut être suivi d'un passage (219) permettant au piston (207) de se libérer des contraintes exercées par ledit moyen d'appui (209).

Le piston peut comprendre une ou plusieurs rampe et/ou un ou plusieurs passage. Au moins une rampe peut avoir une inclinaison entre 0 et 90°. Dans un mode réalisation, ladite inclinaison peut être comprise entre 0 et 45°, préférentiellement, entre 10 et 30°.

Ledit piston (207) comprend au moins deux positions stationnaires :
- une première position où le piston (207) est situé à une distance (d2) égale à A. Dans cette position, le moyen d'appui (209) se trouve au début de la rampe.
- une deuxième position où le piston (207) est situé à une distance (d2) égale à B. Dans cette position, le moyen d'appui (209) coopère avec un seuil permettant au piston de se maintenir à cette position.

Le système a plusieurs avantages :
- Pas besoin d'alimenter le moteur pour maintenir la valve ouverte (3ème état stable).
- L'actionneur ne va pas chauffer lorsque que ce dernier maintient une position.
- Bruit en fonctionnement faible.
- Course importante.

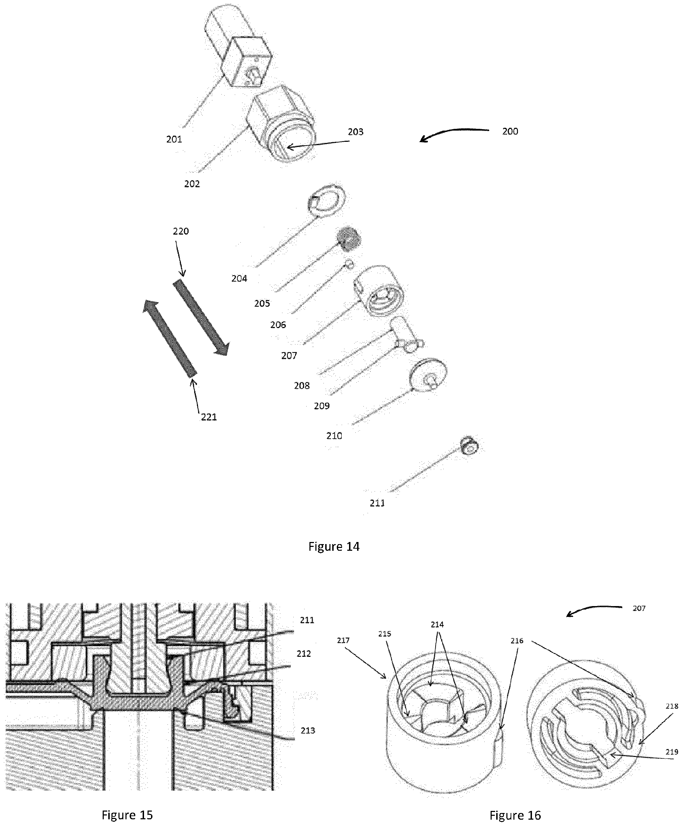

Dans un mode de réalisation préféré tel que montré sur la figure 16, le piston (207) dispose d'au moins une rampe (214) (de préférence deux ou plus) et le moyen d'appui peut être un axe transversale adapté pour coopérer au moins temporairement avec ladite aux moins une rampe.

Aussi, lorsque le piston comprend deux rampes positionnées de façon symétrique par rapport au centre de l'axe du rotor, pour un tour complet du rotor, le piston peut être à deux reprises en deuxième position et première position. Le piston peut comprendre un passage (219) permettant une transissions rapide de la deuxième position à la première position, ledit passage peut s'étendre en dessous de la première position. Cela permet :
- d'obtenir deux positions du piston (première et troisième position) sans actionnement de l'actionneur,
- faciliter l'assemblage,
- empêcher le rotor (ou le moteur) de tourner lorsque le piston est en troisième position, ce qui rend impossible tout changement de position lorsque la cassette n'est pas chargé dans l'appareil.

Selon un mode de réalisation, la rampe est suivit d'au moins un passage, préférentiellement après un seuil.

Dans un mode réalisation, ledit actionneur comprend en outre au moins un moyen de compression (205) qui exercent une force contre le piston (207). Le moyen d'appui et la rampe coopère afin de mouvoir le piston selon un même axe que la force exercée par le moyen de compression mais de sens opposé.

Dans un mode de réalisation, ledit moyen de compression tend à repousser le piston (par rapport à l'actionneur) (c'est-à-dire en direction (220) de l'extrémité distale du piston (217)) alors que les moyens d'appui et la rampe contraignent le piston à se rapprocher de l'actionneur. Dans ce cas, A > B. Dans un autre mode de réalisation, ledit moyen de compression tend à rapprocher le piston de l'actionneur alors que les moyens d'appui et la rampe contraignent le piston à s'éloigner de l'actionneur. Dans ce cas, A < B.

Dans un mode réalisation, l'actionneur a pour objectif d'entrainer un élément d'un appareil tel que décrit dans le présent document. Il peut s'agir par exemple d'une valve de la cassette. La suite de la description décrit ce mode de réalisation mais il va de soi que l'invention ne se limite pas à ce mode de réalisation.

Ainsi, ledit piston (207) comprend au moins deux positions :
- une première position où le téton (211) du piston (207) est couplé à la valve (212) (illustration en figure 15) d'une cassette tel que décrit précédemment. Le piston (207) n'étant pas contraint par le moyen d'appui (209), maintient la valve (212) en position fermée contre le siège de valve (213). Ici, d2 est égal à A.
- une deuxième position où le téton (211) du piston (207) est couplé à la valve (212). Le piston (207) est contraint par le moyen d'appui (209), déplaçant l'ensemble piston/téton en direction (221) du moteur (201). Lorsque que ledit moyen d'appui (209) arrive au seuil (215) se trouvant au sommet de la rampe, le piston (207) est en deuxième position et la valve (212) en position ouverte. Ici, d2 est égal à B.

Dans un mode de réalisation, le piston connait une troisième position, où le téton (211) du piston (207) est découplé à la valve (212). Un moyen de compression (205 exerce une force contre le piston, déplaçant ledit piston vers une troisième position plus éloigné du moteur que la première et deuxième position. Il peut s'agir du même moyen de compression décrit ci-dessus ou un moyen de compression distinct. Ici, d2 est égal à C. Dans ce mode de réalisation C > A > B. L'intérêt de cette troisième position est de garantir une pression d'occlusion suffisante lorsque le piston est couplé à la valve en première position. Dit autrement, lorsque le piston est couplé à la valve, le piston exerce une force contre la valve afin d'assurer la fermeture de la valve quand le piston est en première position.

Dans mode de réalisation, l'actionneur comprend un élément de fixation à son support comprenant un moyen de compression exerçant une force en direction de l'extrémité distale du piston et ayant la même fonction décrite ci-dessus.

Lesdits moyens de compression (205) peuventt être un ressort, une lame élastique, un élastique ou un matériau à mémoire de forme. Ledit moyen de compression (205) peut exercer une force de 0 à 6N, préférentiellement entre 5 et 6N.

L'actionneur (200) est conçu dans le but ne pas consommer d'énergie lors du maintien d'une position stationnaire. Le moyen d'appui (209) est conçu pour glisser ou rouler sur la rampe afin d'atteindre une position. Lorsque moyen d'appui (209) s'arrête sur un seuil, ledit seuil est façonné de manière à ce que l'ensemble soit à l'équilibre. Le seuil (215) au sommet de la rampe (214) est directement suivi d'un passage (219) permettant au piston de passer rapidement d'une deuxième position à une première position en consommant un minimum d'énergie. Ledit passage permet de passer d'une position à l'autre avec une faible quantité d'énergie. Autrement dit, l'énergie consommée par l'actionneur pour passer de la première position à la deuxième position est supérieure à l'énergie consommée par l'actionneur pour passer de la deuxième position à la première position. Le passage (219) peut être une rampe ayant une pente élevée et/ou de sens opposé à la pente de la rampe. Ainsi, le moyen d'appui parcourt une plus faible distance pour passer de la deuxième position à la première qu'inversement.

Optionnellement, le piston (207) comprend plusieurs seuils afin d'avoir des positions intermédiaires de repos.

Dans un mode de réalisation, le moteur comprend un réducteur de couple entre le moteur et le rotor des moyens interposés. Ledit réducteur de couple peut être conçu de manière à ce que le moteur puisse faire tourner le rotor mais que le rotor ne puisse pas faire tourner le moteur. Dit autrement, le réducteur de couple peut grâce à sa conception empêcher ou limiter ou freiner tout mouvement du rotor qui ne serait pas dû par le moteur.

Dans un mode de réalisation, le réducteur de couple peut être conçu de manière à ce que l'actionneur puisse maintenir n'importe quelle position lorsque le moteur est à l'arrêt (alimenté ou non). Ainsi, l'actionneur peut comprendre un nombre limité de seuil tel que décrit précédemment mais un nombre illimité de position qui peuvent être maintenu grâce au réducteur de couple sans que l'actionneur soit alimenté en courant. Un tel actionneur peut être adapté pour coopérer avec une valve proportionnelle d'une cassette à distribution de fluide. Ainsi, grâce à cette conception, l'actionneur peut permettre l'écoulement d'un fluide proportionnellement au besoin du traitement.

Dans un mode de réalisation, le piston (207) ne comprend aucun seuil mais uniquement des positions maintenables grâce au réducteur de couple comme décrits précédemment. Ce piston comprend ainsi au moins une rampe et optionnellement un passage. Le réducteur de couple permet à l'actionneur de maintenir une position donné permettant l'ouverture de 0% à 100% d'une valve (par exemple une valve proportionnelle).

Dans un mode de réalisation, le piston comprend au moins une rampe inférieure et une rampe supérieure. Lesdites rampes étant adaptées pour qu'au moins un moyen d'appui (209) puisse se déplacer entre lesdites rampes. Lesdites rampes peuvent au moins en partie être parallèle l'une par rapport à l'autre.

Dans un mode réalisation préféré, au moins un actionneur est compris dans un système d'actionnement qui comprend un contrôleur et au moins un moyen d'alimentation en énergie. Ledit système est conçu pour déplacer aux moins un piston d'une deuxième position à une première position et vice versa en consommant une faible quantité d'énergie. Lesdits moyens d'alimentation en énergie peuvent être une alimentation extérieure et/ou un moyen de stockage d'énergie. Ledit moyen de stockage d'énergie peut être utilisé par le système lorsque ladite alimentation extérieure n'est plus opérante ou insuffisante. Ainsi, en cas de coupure de courant, la valve passera de l'état ouvert à fermer grâce à l'utilisation dudit moyen de stockage d'énergie qui peut être une super capacité ou d'une batterie.

Selon un mode de réalisation divulgué par la figure 14, l'actionneur (200) peut être composé :
- d'un moteur (201)
- d'une enveloppe rigide (202), à l'intérieur de laquelle est disposé :
   ∘ un capteur(204) fixé au moteur avec ladite enveloppe (202),
   ∘ un moyen de compression (205),
   ∘ un piston (207) dans lequel est fixé élément (206) adapté pour coopérer avec ledit capteur (204) ,
   ∘ un moyen d'appui (209) fixé directement ou indirectement au rotor (208) du moteur (201),
   ∘ un élément (210) fixé sur l'extrémité distale (217) du piston permet de fixer un téton (211) qui sera couplé à une valve.

Le piston (207) et l'enveloppe rigide (202) comprennent des moyens de guidage (203, 216) afin d'éviter que le piston ne tourne avec le rotor (208) du moteur.

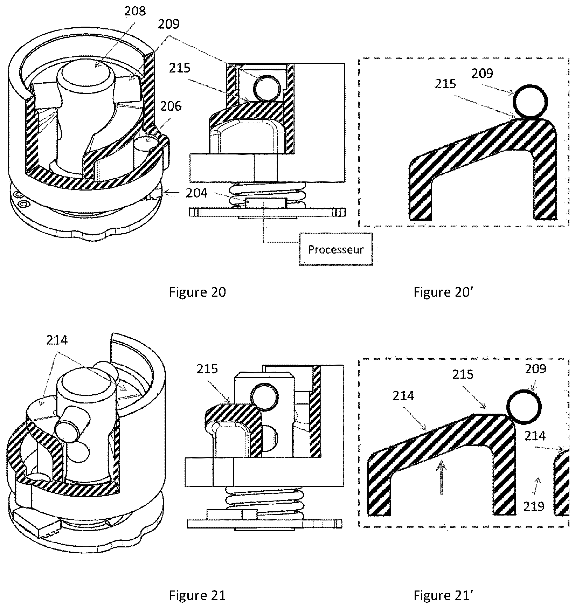

Les figures 17, 18, 19, 20 et 21 montrent le piston en différentes position (la valve et le téton ne sont représentés sur ces figures) :
∘ Figure 17, la valve n'est pas couplée au téton. Le piston (207) est en troisième position avec le moyen de compression (205) détendu. Le moyen d'appui (209) est dans le passage (219) et n'exerce aucune force contre la rampe (214).
∘ Figure 18, la valve est couplée au téton. Le piston (207) est en première position, le moyen de compression (205) exerce une pression contre le piston (207) afin de garantir la position fermée de la valve. Préférentiellement, le moyen d'appui (209) et la rampe (214) n'exercent aucune contrainte. La figure 18' permet de mettre en évidence deux modes de réalisation distinct du seuil (215) au sommet de la rampe. Ainsi, selon un mode de réalisation, ledit seuil peut avoir différente forme, il peut être plat ou plus ou moins façonné pour une plus grande coopération avec le moyen d'appui (209) lorsque ce dernier se trouve à proximité et/ou sur le seuil (215).
∘ Figure 19, la valve est couplée au téton. Le rotor (208) est en mouvement pour permettre au piston (207) de passer de la première position à la deuxième position. Le moyen d'appui (209) effectue sa course sur la rampe (214) et contraint le piston (207) à se rapprocher du moteur (ouverture de la valve) et compresse le moyen de compression (205).
∘ Figure 20, la valve est couplée au téton. Le piston (207) est en deuxième position, le moyen d'appui (209) s'arrête sur le seuil (215) au sommet de la rampe. Le moyen de compression (205) est comprimé. La valve est ouverte. La position est stable sans contribution du moteur (201). Dans un mode de réalisation, l'actionneur comprend un capteur conçu pour connaitre la position relative du piston (207). Le mode de réalisation exposé à travers la figure 20 expose un capteur (204) à effet hall coopérant avec un aimant (206) logé dans le piston. Le capteur (204) peut être un capteur de déplacement linéaire comprenant une tige ou un encodeur ou tous éléments (206) étant adaptés pour coopérer avec ledit capteur (204). En outre, grâce au processeur relié au capteur (204), il est possible de connaître ou contrôler la position dudit piston (207).
∘ Figure 21, la valve est couplée au téton. Le rotor (208) est en mouvement faisant passer le moyen d'appui (209) dans le passage (219). Le piston passe instantanément de la deuxième position à la première position grâce au moyen de compression (205) qui repousse le piston (207) en direction de l'extrémité distale du piston (217). La valve se ferme.

### Système de commande et de contrôle d'un actionneur

Dans un mode de réalisation divulgué par les figures 32, 32' et 33, un système de commande (800) comprend un actionneur linaire comprenant une partie mobile (801) et une partie fixe (804) ainsi que des éléments de contrôle et de commande (802, 803, 805). Les éléments de contrôle et de commande sont adaptés pour connaître la position de la partie mobile (801) par rapport à la partie fixe (804) et pour commander l'actionneur linéaire.

La figure 32 expose l'actionneur en une position A et la figure 32' expose l'actionneur en une position B. L'élément de commande (805) commande l'actionneur dont la partie fixe (804) peut comprendre le moyen d'entrainement (par exemple un moteur). La partie mobile (801) peut être adaptée pour coopérer avec par exemple une valve d'un système de distribution de fluide. Ainsi, la position A pourrait correspondre à la position fermée de la valve à commander et la position B pourrait correspondre à la position ouverte de ladite valve, toutefois l'invention ne se limite à la commande d'ouverture et de fermeture d'une valve d'un système de distribution de fluide et le nombre de position peut être limité ou illimité.

Les éléments 1 (802) et 2 (803) du capteur sont conçus pour coopérer et déterminer au moins une position. Il peut s'agir d'un capteur de déplacement capacitif ou inductif (LVDT,...), un capteur électromagnétique (capteur à effet hall), capteur ultrason, infrarouge, optique, laser, mécanique ou micro-onde (liste non limitative). Dans notre exemple et pour faciliter la compréhension, nous utiliserons un capteur à effet hall. Ainsi, l'élément 1 (802) est un aimant permanent (dans ce cas, il n'est pas relié au processeur) (805) et l'élément 2 (803) est un capteur à effet hall relié au processeur. L'aimant permanent crée un champ électromagnétique dont le capteur (803) mesure l'intensité. En particulier le capteur (803) permet de détecter la variation du champ magnétique induit par l'aimant permanent (802) lorsque qu'il se déplace.

De préférence, l'aimant permanent (802) est rigidement fixé de manière définitive à la partie mobile (801) de l'actionneur et le capteur (803) est rigidement fixé de manière définitive à la partie fixe (8004) de l'actionneur (ou inversement). Ainsi, lorsque la partie mobile se déplace l'aimant permanent (802) se rapproche ou s'éloigne du capteur (803) qui mesure ainsi une variation de l'intensité du champ magnétique de l'aimant permanent (802). Idéalement, l'aimant et le capteur sont alignés.

Les données de mesure du capteur (802) sont transmises au processeur pour traiter le signal. Normalement, tous les systèmes de commande doivent être qualifiés afin de déterminer à l'avance l'intensité correspondant à chaque position. Dit autrement, généralement, le capteur détecte des seuils de valeur prédéterminés correspondant à des positions respectives déterminées à l'avance. Or, ce travail de qualification (par exemple calibration qui doit être effectué à tous les actionneurs) est long et onéreux. Afin d'éviter ce travail de qualification, l'invention divulgue l'utilisation d'un processeur qui effectue un traitement du signal pour déterminer au moins une position de l'actionneur. Ainsi, l'invention permet par exemple de ne pas effectuer de calibration.

Le graphique supérieur de la figure 33 permet de mettre en évidence que la valeur absolue (l'intensité du champ électromagnétique de l'aimant mesuré par le capteur) peut être très différente d'un actionneur à l'autre. En effet, les capteurs des actionneurs 1 et 2 ne relèvent pas la même intensité (en valeur absolue) alors que leur position est identique. Ainsi, il serait difficile et peu fiable voire impossible de déterminé la position de ces actionneurs en fonction d'une unique valeur seuil.

Le système de commande (800) comprend un processeur (805) qui utilise un modèle mathématique tenant compte de la dérivée de la valeur absolue. Dans ce présent document, la valeur absolue est la valeur mesurée par le capteur (803), il correspond à l'intensité du champ magnétique. La courbe de la valeur absolue est représentée par le graphique supérieur de la figure 33. La dérivée de la valeur absolue est le coefficient directeur de la courbe dessinée par la valeur absolue. Autrement dit, la dérivée permet de connaître la pente de la variation du champs magnétique lorsque l'aimant se déplace par rapport au capteur. Cette dérivée est représentée par la courbe du graphique du milieu de la figure 33. Ainsi, cette dérivée permet de connaître le sens du déplacement de la partie mobile (801) de l'actionneur par rapport à sa partie fixe (804).

Le système de commande comprend ainsi un processeur utilisant un modèle mathématique qui tient compte de la dérivée du signal. Grâce à ce modèle mathématique, il est possible de savoir quand l'actionneur a atteint une position ou un seuil tel que décrit dans le chapitre divulguant l'actionneur linéaire. En effet, lorsque l'actionneur déplace sa partie mobile (801) la dérivée première est supérieure ou inférieur à 0 mais lorsque l'actionneur ne déplace pas sa partie mobile (801), sa dérivée première est substantiellement égale à 0. Dans notre exemple et préférentiellement, lorsque l'aimant (802) s'éloigne du capteur (803) la dérivée première est négative et inversement lorsque l'aimant se rapproche, la dérivée première est positive.

Le système peut en outre comprendre un modèle mathématique pour déterminer quand la partie mobile se déplace et quand elle reste immobile. Ce deuxième modèle mathématique tient compte de la dérivée seconde de la valeur absolue. Grâce à ce deuxième modèle mathématique, le système sait quand la partie mobile change son comportement (mobile ou immobile).

Dans un mode de réalisation, le système de commande comprend un actionneur comprenant au moins une rampe et au moins un seuil (par exemple, un actionneur linéaire tel que décrit dans le présent document), un processeur adapté pour commander l'actionneur et pour traiter le signal selon au moins un modèle mathématique.

Un premier modèle mathématique prend en compte la dérivée première de la valeur absolue mesurée par le capteur (803). Le processeur peut utiliser ce premier modèle mathématique afin de savoir dans quel sens la partie mobile (801) se déplace. Lorsque le dérivé premier est proche de 0, le système de commande sait que l'actionneur a atteint un seuil.

Un deuxième modèle mathématique prend en compte la dérivée seconde de la valeur absolue mesurée par le capteur (803). Le processeur peut utiliser le deuxième modèle mathématique afin de savoir quand un seuil est atteint et/ou quand la partie mobile est immobile ou se déplace. Le graphique inférieur de la figure 33 représente le signal résultant du deuxième modèle mathématique. Lorsque que le signal est égal à la valeur f, cela signifie que l'actionneur maintient une position ou est sur un seuil. Lorsque le signal est égal à la valeur d, signifie que la partie mobile (801) se déplace. Ainsi, grâce à ce deuxième modèle mathématique, le système n'a pas besoin de la valeur absolu. Lorsque le système de commande alimente l'actionneur pour mouvoir sa partie mobile (801), le deuxième modèle mathématique permet de savoir quand l'actionneur a atteint une position. Autrement dit, lorsque l'actionneur continu son actionnement (par exemple, il fait tourner le moyen d'appui (209)) mais que la partie mobile ne bouge plus, grâce à la dérivée première et/ou seconde, le processeur est capable de savoir que le moyen d'appui a atteint un seuil. Ainsi, quand le processeur ordonne à l'actionneur un changement de position, un modèle mathématique permet au processeur de savoir quand le seuil est atteint et ainsi ordonne l'actionneur de s'arrêter.

Ainsi, ledit système de commande est adapté pour déterminer au moins une position atteinte par la partie mobile (801) de l'actionneur indépendamment des caractéristiques du capteur utilisé. Ledit système de commande est adapté pour commander l'arrêt de l'actionneur à au moins une position atteinte par la partie mobile (801) de l'actionneur indépendamment des caractéristiques du capteur utilisé.

Dans un mode de réalisation, le système de commande comprend un actionneur comprenant au moins deux positions distinctes. L'actionneur comprend au moins un seuil définissant une position et au moins une rampe permettant de changer de position. L'actionneur est entrainé par un moteur conçu pour tourner préférentiellement dans un seul sens de sorte qu'il passe d'une position à une autre de façon séquentielle et dans un ordre préétabli. Préférentiellement, l'actionneur est adapté pour revenir à sa position de départ en effectuant au moins une révolution partielle. En outre, le processeur comprend un modèle mathématique qui tient compte de la dérivée seconde de la valeur absolue mesurée par ledit capteur. Ledit processeur comprend une mémoire qui contient la séquence des positions de sorte que ledit système n'a pas besoin de connaitre le dérivé premier de la valeur absolue pour connaître la position de l'actionneur. Il suffit à l'actionneur d'effectuer une révolution pour connaitre avec précision sa position, par exemple, lors de la mise en route du système.

### Dispositif d'entrainement utilisé pour les pompes péristaltiques :

Dans un mode de réalisation, le système de traitement peut comprendre un dispositif d'entrainement utilisé pour les pompes péristaltiques. Ledit dispositif d'entrainement divulgué par le présent document peut être également utilisé par diverse pompe péristaltique et/ou système de distribution de fluide comprenant une pompe péristaltique.

L'invention divulgue un moyen de corriger les erreurs de tolérance de l'axe entrainant les pompes péristaltiques. Ladite invention, présentée en figure 22 et 23, est un dispositif d'entrainement (300) qui comprend un axe flottant (301) entrainé par un moyen d'entrainement (303) fixé à un rotor (310) d'un moteur électrique (non schématisé). Ledit axe flottant (301) comprend un ensemble solidaire base(311)/couvercle(302) renfermant une cavité à l'intérieur de laquelle ledit moyen d'entrainement (303) est au moins partiellement circonscrit. Ledit moyen d'entrainement (303) comprend un corps rigide façonné de manière à coopérer avec les parois (309, 309') de ladite cavité (313) afin de permettre une liberté restreinte de l'axe flottant (301) par rapport à l'axe dudit rotor (310). Une vis (307) peut permettre de fixer le moyen d'entrainement (303) audit rotor (310).

La cavité comprend au moins un élément de coopération (312) qui permet audit moyen d'entrainement (303) de transmettre un mouvement de rotation audit axe flottant. Préférentiellement, ledit élément de coopération (312) est une ouverture bornée par deux éléments durs (308) et à travers lesquelles un axe (306) vient se loger perpendiculairement. L'espace entre les deux éléments durs (308) est raisonnablement supérieur au diamètre de l'axe (306).

Les éléments durs (308) et/ou l'axe (306) peuvent être fabriqué à partir de métaux dure tel que cobalt, tungstène, vanadium, chrome, manganèse, nickel, titane, germanium, gallium, bismuth, iridium, lithium, magnésium, molybdène, strontium, rubidium ou palladium. Dans un mode de réalisation, les éléments durs (308) ont une dureté plus importante que l'axe (306). Les éléments durs (308) et/ou l'axe (306) peuvent recevoir un traitement pour accroitre leur dureté, par exemple de l'oxyde de zirconium ou un de ses alliages.

Dans un mode de réalisation, le corps (304) dudit moyen d'entrainement (303) peut être de forme sphérique parfaitement ronde ou partiellement aplatie. Dans autre mode de réalisation, ledit corps (304) forme un galet comprenant trois faces. Deux des trois faces s'opposent et sont reliées entre elles par l'intermédiaire de la troisième face qui est courbe. Selon le plan X-Z, ledit galet forme un cercle formé par ladite face courbe. La liaison entre au moins une des deux faces qui s'opposent avec la face courbe peut être arrondie selon le plan X-Y. Les faces qui s'opposent peuvent être substantiellement planes et/ou substantiellement parallèles l'une par rapport à l'autre.

Dans un mode de réalisation, la cavité (313) comprend des parois lisses (309, 309'). Préférentiellement, la paroi supérieure (309) et/ou la paroi inférieure (309') de ladite cavité (313) sont de forme au moins partiellement conique. Les surfaces des parois supérieures (309) et inférieures (309') peuvent être planes ou incurvées.

Dans mode de réalisation, selon le plan X-Y, le cône de la paroi lisse (309) est défini selon un angle compris entre 0 et 90°, préférentiellement entre 5 et 30°. Le cône de la paroi lisse opposée (309') est défini selon un angle compris -0 et -90°, préférentiellement entre -5 et -30°. Les angles des deux cônes partiels peuvent être égaux ou différents.

Les parois lisses (309, 309') sont adaptées pour coopérer avec les extrémités du corps (304) de sorte que l'axe flottant (301) puisse se mouvoir selon au moins des trois axes X, Y ou Z et/ou subir des mouvements de tangage. Par exemple, selon l'axe Y, l'axe flottant (301) peut subir un mouvement de tangage de +/- 10°, préférentiellement inférieur à +/- 5°.

Dans un mode de réalisation, le moyen d'entrainement (303) comprend un axe longitudinal (305) et l'axe flottant (301) comprend une deuxième cavité (313') qui s'étend le long de l'axe flottant. L'axe longitudinal (305) s'insère à l'intérieur de la deuxième cavité (313') afin de restreindre les mouvement de tangage de l'axe flottant (301).

De manière générale et préférentiellement, les dimensions des éléments formant le moyen d'entrainement (303, 306, 305) sont raisonnablement plus petits que les éléments formant l'intérieur de l'axe flottant (301, 302, 313, 312).

Dans un mode de réalisation, le système d'entrainement (300) comprend un axe d'entrainement formé d'une seule pièce qui s'étend selon l'axe Y et qui est adapté pour entrainer au moins un galet (320) d'un système de pompe péristaltique. Ledit galet est adapté pour écraser un tube flexible (non représenté) contre une paroi (non représenté). Selon les figures 22 et 23, l'axe d'entrainement est représenté par l'axe flottant (301). Autrement dit, l'axe d'entrainement peut être l'axe flottant et/ou confondu avec une partie du système d'entrainement. Selon les figures 22 et 23, l'axe d'entrainement peut être de forme cylindrique et un comprendre une extrémité libre biseautée (ici le terme extrémité libre s'oppose à l'extrémité opposé qui est lié au directement ou indirectement au moteur). Le cylindre de l'axe d'entrainement forme un cercle selon un plan X-Z.

Dans mode de réalisation, ledit axe d'entrainement est formé d'une seule pièce composée d'au moins deux cylindres différents définis par le même axe (autrement dit centre du cylindre) mais ayant des diamètres différents. Ainsi, selon le plan X-Z, l'axe d'entrainement peut former au moins deux cercles parallèles mais de tailles différentes. En outre, l'axe d'entrainement peut comprendre trois cylindres dont un seul est de diamètre différent. Le cylindre définit par le diamètre le plus petit peut être agencé entre les deux cylindres de diamètre égaux. Lesdits cylindres de diamètre plus important peuvent présenter des surfaces traitées de manière à améliorer la coopération avec les galets de la pompe péristaltique.

Cette construction de l'axe d'entrainement à trois cylindres est particulièrement adaptée et avantagé pour l'utilisation d'un galet en forme de H, tel que le galet (320) de la figure 23. Le galet est compris dans un système de pompe péristaltique et vient écraser un tuyau flexible. La fonction d'écrasement est de préférence effectuée par une partie rigide (322) du galet (320). Ledit galet (320) est un cylindre formant un cercle sur le plan X-Z et comprend un axe (321) au centre de ce cylindre qui s'étend selon l'axe Y, une partie rigide (322) et au moins une partie flexible (323) qui est déformable. La partie flexible est adaptée pour coopérer avec l'axe d'entrainement.

Préférentiellement, les cylindres de diamètre plus important entre en contact avec les parties flexible (323) du galet (320) et entraine le galet (320). Lorsque l'axe d'entrainement est en contact avec au moins une partie flexible, ladite partie flexible (323) peut se déformer afin d'améliorer de coopération entre ces deux éléments et/ou pour ajuster les erreurs de tolérance de chacun des éléments. De préférence, le galet comprend un partie rigide (322) en son centre et deux parties flexible (323) aux extrémités selon l'axe Y.

Un système de distribution de fluide comprenant un axe flottant (301), un axe d'entrainement à trois cylindres et/ou un galet à parties rigide et flexible permet d'améliorer substantiellement la coopération galet / axe d'entrainement et de corriger les erreurs de tolérance des différents éléments du système.

### Moyen d'amortissement des pics de pression :

Dans un mode de réalisation, le système de traitement comprend au moins un moyen d'amortissement des pics de pression.

La figure 24 montre le signal de pression mesurée près de l'entrée d'une pompe péristaltique. Sur la première courbe (401), il est possible d'observer l'oscillation de la pression alors que la deuxième courbe (402) représente la valeur moyenne de cette pression. L'objectif de l'amortisseur est de réduire l'amplitude des oscillations de la première courbe (401).

Un tel moyen d'amortissement peut être installé dans un ou plusieurs chemins fluidiques d'un système de distribution tel que décrit précédemment. Préférentiellement, ce moyen d'amortissement est intégré dans une cassette.

Que ce soit en dialyse péritonéale ou en thérapie d'épuration extra-rénale continue, chaque traitement dispose d'une ou plusieurs configurations possibles. Or, le phénomène de pics de pression peut être amplifié ou atténué par divers éléments qui varient en fonction desdites configurations. Pour rappel, l'un des objectifs de l'invention est de permettre un usage simple du système. La reproductibilité est ainsi un élément important car l'opérateur (généralement l'infirmier) doit pouvoir s'assurer du bon fonctionnement du système sans être obligé de prendre en compte les caractéristique des différents éléments.

Le système de distribution tel que dévoilé dans ce document est une cassette à travers laquelle des fluides s'écoulent dans trois chemins fluidiques. Préférentiellement, ces fluides sont propulsés par des pompes péristaltiques. La reproductibilité, la précision et le confort du patient sont des éléments importants. Ainsi préférentiellement, au moins un moyen d'amortissement est intégré dans ladite cassette. Pour être le plus efficace possible, ledit moyen d'amortissement doit être placé le plus proche possible du système de pompage.

Dans un mode de réalisation schématisé en figure 25, le chemin fluidique comprend des parois (404) délimitant ledit chemin. Ces parois peuvent être les parois rigides de la cassette. Une section du chemin fluidique est recouverte d'une membrane flexible (403) qui remplace alors ladite paroi (404). Grâce à l'élasticité de ladite membrane flexible (403), les pics de pression sont sensiblement atténués, absorbés par la déformation de la membrane (403). L'absorption de ces pics est fonction de la taille et des caractéristique élastiques de la membrane.

Dans un autre mode de réalisation schématisé en figure 26, le chemin fluidique comprend également des parois délimitant ledit chemin mais une section est remplacée par une cavité remplie d'un fluide compressible tel que de l'air. Cet air peut avoir été piégé (environ 1mL) lors du priming du système. Ainsi, le volume de l'air est plus ou moins compressé durant le pompage, absorbant l'énergie des pics de pression. Ce mode de réalisation est particulièrement efficace car il requiert peu d'énergie pour son fonctionnement et ledit fluide absorbe très rapidement les pics de pression. De préférence, la cavité ou l'entrée de la cavité est conçu pour éviter que le fluide compressible de s'échappe totalement. Autrement dit, la cavité est conçue pour éviter que le fluide qui s'écoule ne remplace le fluide compressible.

Préférentiellement, ledit moyen d'amortissement est placé en amont du mécanisme de pompage.

## Revendications

1. Dispositif d'entrainement pour une pompe péristaltique d'un dispositif médical comprenant :
- un moyen d'entrainement (303) fixé à un rotor (310) d'un moteur et
- un axe flottant (301) ayant un axe de rotation entrainé par le moyen d'entrainement (303) ;
dans lequel l'axe flottant (301) comprend un ensemble solidaire base (311) / couvercle (302) formant une cavité (313) à l'intérieur de laquelle le moyen d'entrainement (303) est au moins partiellement circonscrit,
dans lequel ledit moyen d'entraînement (303) comprend un corps rigide (304) façonné de manière à coopérer avec des parois internes (309, 309') de ladite cavité (313) afin de permettre une liberté restreinte de l'axe de rotation de l'axe flottant (301) par rapport à l'axe dudit rotor (310) de manière à relaxer les tolérances de fabrication de la pompe péristaltique.

2. Dispositif selon la revendication 1, dans lequel ladite la cavité (313) comprend au moins un élément de coopération (312) configurer pour permettre au moyen d'entraînement (303) d'exercer un mouvement de rotation audit axe flottant (301).

3. Dispositif selon la revendication 2, dans lequel le moyen d'entrainement (303) comprend un axe transversal (306) configuré pour engager l'élément de coopération (312) de manière à appliquer un mouvement de rotation à l'axe flottant (310).

4. Dispositif selon la revendication 2, dans lequel l'élément de coopération (312) est constitué d'une composition comprenant un élément parmi la liste suivante : cobalt, tungstène, vanadium, chrome, manganèse, nickel, titane, germanium, gallium, bismuth, iridium, lithium, magnésium, molybdène, strontium, rubidium et palladium.

5. Dispositif selon la revendication 3, dans lequel axe transversal (306) est constitué d'une composition comprenant un élément parmi la liste suivante : cobalt, tungstène, vanadium, chrome, manganèse, nickel, titane, germanium, gallium, bismuth, iridium, lithium, magnésium, molybdène, strontium, rubidium et palladium.

6. Dispositif selon l'une des revendications précédentes, dans lequel le corps dudit moyen d'entraînement (303) comprend des bords arrondis lisses et la cavité (313) comprend des parois lisses.

7. Dispositif selon l'une des revendications précédentes, dans lequel les parois internes de ladite cavité sont de forme conique.

8. Dispositif selon l'une des revendications précédentes, dans lequel le moyen d'entraînement (303) comprend un axe longitudinal (305) et l'axe flottant (301) comprend une deuxième cavité (313') qui s'étend le long de l'axe flottant (301).

9. Dispositif selon la revendication 8, dans lequel l'axe longitudinal (305) est configuré pour s'insérer à l'intérieur de la deuxième cavité (313') afin de restreindre les déplacements latéraux de l'axe flottant (301).

10. Dispositif selon l'une des revendications précédentes, dans lequel les dimensions des éléments formants le moyen d'entraînement (303) sont au moins plus petit que les éléments intérieurs de l'axe flottant (301).

11. Dispositif selon l'une des revendications précédentes, dans lequel les parois internes (309, 309') de la cavité (313) sont configurées pour coopérer avec des parois du moyen d'entrainement (303) pour permettre à l'axe flottant (301) de se déplacer linéairement par rapport au moyen d'entrainement (303).

12. Dispositif selon l'une des revendications précédentes, dans lequel les parois internes (309, 309') de la cavité (313) sont configurées pour coopérer avec des parois du moyen d'entrainement (303) pour permettre à l'axe de rotation de l'axe flottant (301) de subir des mouvements de tangage par rapport à l'axe moteur compris entre +/- 10° ou +/- 5°.

13. Système de pompe médicale comprenant :
- une pompe péristaltique ; et
- un dispositif d'entrainement selon la revendication 1 configuré pour coopérer avec la pompe péristaltique.

14. Dispositif selon la revendication 13, dans lequel la pompe péristaltique comprend au moins un galet et un tube flexible.

## Patentansprüche

1. Antriebsvorrichtung für eine peristaltische Pumpe einer medizinischen Vorrichtung, umfassend:
- ein Antriebsmittel (303), das an einem Rotor (310) eines Motors fixiert ist, und
- eine schwimmende Achse(301) mit einer von dem Antriebsmittel (303) angetriebenen Rotationsachse;
wobei die schwimmende Achse (301) eine fest verbundene Anordnung Basis (311)/Deckel (302) umfasst, die einen Hohlraum (313) bildet, in den das Antriebsmittel (303) mindestens teilweise einbeschrieben ist,
wobei das Antriebsmittel (303) einen starren Körper (304) umfasst, der so gestaltet ist, dass er mit Innenwänden (309, 309') des Hohlraums (313) zusammenwirkt, um eine eingeschränkte Freiheit der Rotationsachse der schwimmenden Achse (301) in Bezug auf die Achse des Rotors (310) zu ermöglichen, so dass die Fertigungstoleranzen der peristaltischen Pumpe ausgeglichen werden.

2. Vorrichtung nach Anspruch 1, wobei der Hohlraum (313) mindestens ein Zusammenwirkelement (312) umfasst, das dazu ausgestaltet ist, dem Antriebsmittel (303) zu ermöglichen, eine Rotationsbewegung auf die schwimmende Achse (301) auszuüben.

3. Vorrichtung nach Anspruch 2, wobei das Antriebsmittel (303) eine Querachse (306) umfasst, die dazu ausgestaltet ist, in das Zusammenwirkelement (312) eingeführt zu werden, so dass eine Rotationsbewegung auf die schwimmende Achse (310) angewandt wird.

4. Vorrichtung nach Anspruch 2, wobei das Zusammenwirkelement (312) aus einer Zusammensetzung besteht, die ein Element aus der folgenden Liste umfasst: Cobalt, Wolfram, Vanadium, Chrom, Mangan, Nickel, Titan, Germanium, Gallium, Bismut, Iridium, Lithium, Magnesium, Molybdän, Strontium, Rubidium und Palladium.

5. Vorrichtung nach Anspruch 3, wobei die Querachse (306) aus einer Zusammensetzung besteht, die ein Element aus der folgenden Liste umfasst: Cobalt, Wolfram, Vanadium, Chrom, Mangan, Nickel, Titan, Germanium, Gallium, Bismut, Iridium, Lithium, Magnesium, Molybdän, Strontium, Rubidium und Palladium.

6. Vorrichtung nach einem der vorhergehenden Ansprüche, wobei der Körper des Antriebsmittel (303) glatte abgerundete Kanten umfasst und der Hohlraum (313) glatte Wände umfasst.

7. Vorrichtung nach einem der vorhergehenden Ansprüche, wobei die Innenwände des Hohlraums konisch geformt sind.

8. Vorrichtung nach einem der vorhergehenden Ansprüche, wobei das Antriebsmittel (303) eine Längsachse (305) umfasst und die schwimmende Achse (301) einen zweiten Hohlraum (313') umfasst, der sich entlang der schwimmenden Achse (301) erstreckt.

9. Vorrichtung nach Anspruch 8, wobei die Längsachse (305) dazu ausgestaltet ist, in den zweiten Hohlraum (313') eingesetzt zu werden, um die seitlichen Verlagerungen der schwimmenden Achse (301) einzuschränken.

10. Vorrichtung nach einem der vorhergehenden Ansprüche, wobei die Abmessungen der das Antriebsmittel (303) bildenden Elemente mindestens kleiner als die inneren Elemente der schwimmenden Achse (301) sind.

11. Vorrichtung nach einem der vorhergehenden Ansprüche, wobei die Innenwände (309, 309') des Hohlraums (313) dazu ausgestaltet sind, mit Wänden des Antriebsmittels (303) zusammenzuwirken, um der schwimmenden Achse (301) zu ermöglichen, sich in Bezug auf das Antriebsmittel (303) linear zu verlagern.

12. Vorrichtung nach einem der vorhergehenden Ansprüche, wobei die Innenwände (309, 309') des Hohlraums (313) dazu ausgestaltet sind, mit Wänden des Antriebsmittels (303) zusammenzuwirken, um der Rotationsachse der schwimmenden Achse (301) zu ermöglichen, Nickbewegungen in Bezug auf die Motorachse zwischen +/- 10° oder +/- 5° zu erfahren.

13. System einer medizinischen Pumpe, umfassend:
- eine peristaltische Pumpe; und
- eine Antriebsvorrichtung nach Anspruch 1, die dazu ausgestaltet ist, mit der peristaltischen Pumpe zusammenzuwirken.

14. Vorrichtung nach Anspruch 13, wobei die peristaltische Pumpe mindestens eine Rolle und einen Schlauch umfasst.

## Claims

1. Drive device for a peristaltic pump of a medical device, comprising:
- a drive means (303) fixed to a rotor (310) of a motor, and
- a floating shaft (301) having an axis of rotation driven by the drive means (303);
wherein the floating shaft (301) comprises an integral assembly of base (311) and cover (302), forming a cavity (313) within which the drive means (303) is at least partially circumscribed,
wherein said drive means (303) comprises a rigid body (304) shaped in such a way as to cooperate with internal walls (309, 309') of said cavity (313) in order to permit a limited freedom of the axis of rotation of the floating shaft (301) with respect to the axis of said rotor (310) so as to relax the manufacturing tolerances of the peristaltic pump.

2. Device according to Claim 1, wherein said cavity (313) comprises at least one cooperation element (312) configured to allow the drive means (303) to exert a rotational movement on said floating shaft (301).

3. Device according to Claim 2, wherein the drive means (303) comprises a transverse shaft (306) configured to engage the cooperation element (312) in such a way as to apply a rotational movement to the floating shaft (310).

4. Device according to Claim 2, wherein the cooperation element (312) is made of a composition comprising an element from the following list: cobalt, tungsten, vanadium, chromium, manganese, nickel, titanium, germanium, gallium, bismuth, iridium, lithium, magnesium, molybdenum, strontium, rubidium and palladium.

5. Device according to Claim 3, wherein the transverse shaft (306) is made of a composition comprising an element from the following list: cobalt, tungsten, vanadium, chromium, manganese, nickel, titanium, germanium, gallium, bismuth, iridium, lithium, magnesium, molybdenum, strontium, rubidium and palladium.

6. Device according to any one of the preceding claims, wherein the body of said drive means (303) comprises smooth rounded edges and the cavity (313) comprises smooth walls.

7. Device according to any one of the preceding claims, wherein the internal walls of said cavity are of conical shape.

8. Device according to any one of the preceding claims, wherein the drive means (303) comprises a longitudinal shaft (305), and the floating shaft (301) comprises a second cavity (313') which extends along the floating shaft (301).

9. Device according to Claim 8, wherein the longitudinal shaft (305) is configured to fit within the second cavity (313') in order to limit the lateral movements of the floating shaft (301).

10. Device according to any one of the preceding claims, wherein the dimensions of the elements forming the drive means (303) are at least smaller than the inner elements of the floating shaft (301).

11. Device according to any one of the preceding claims, wherein the internal walls (309, 309') of the cavity (313) are configured to cooperate with walls of the drive means (303) in order to allow the floating shaft (301) to move linearly with respect to the drive means (303).

12. Device according to any one of the preceding claims, wherein the internal walls (309, 309') of the cavity (313) are configured to cooperate with walls of the drive means (303) in order to allow the axis of rotation of the floating shaft (301) to undergo pitching movements with respect to the motor axis of between +/- 10° or +/- 5°.

13. Medical pump system, comprising:
- a peristaltic pump; and
- a drive device according to Claim 1, configured to cooperate with the peristaltic pump.

14. Device according to Claim 13, wherein the peristaltic pump comprises at least one roller and a flexible tube.
